(19) Europäisches Patentamt / European Patent Office / Office européen des brevets

(11) **EP 3 890 772 B1**

(12) # EUROPEAN PATENT SPECIFICATION

(45) Date of publication and mention
of the grant of the patent:
**01.04.2026 Bulletin 2026/14**

(21) Application number: **19817408.8**

(22) Date of filing: **05.12.2019**

(51) International Patent Classification (IPC):
***A61K 38/48*** (2006.01)     ***A61P 25/00*** (2006.01)

(52) Cooperative Patent Classification (CPC):
**A61P 25/00; A61K 38/4893; G01N 33/9406;**
G01N 2333/33; G01N 2800/52; Y02A 50/30

(86) International application number:
**PCT/GB2019/053440**

(87) International publication number:
**WO 2020/115490 (11.06.2020 Gazette 2020/24)**

(54) **BOTULINUM TOXIN FOR USE IN THE TREATMENT OF SYMPTOMS OF TRAUMATIC BRAIN INJURY**

BOTULINUMTOXIN ZUR VERWENDUNG BEI DER BEHANDLUNG VON SYMPTOMEN TRAUMATISCHER GEHIRNVERLETZUNGEN

TOXINE BOTULIQUE POUR UTILISATION DANS LE TRAITEMENT DES SYMPTÔMES D'UNE LÉSION TRAUMATIQUE DU CERVEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.12.2018 EP 18210573**

(43) Date of publication of application:
**13.10.2021 Bulletin 2021/41**

(73) Proprietor: **Ipsen Biopharm Limited**
**Wrexham LL13 9UF (GB)**

(72) Inventors:
• **PORRECA, Frank**
**Wrexham Industrial Estate**
**Wrexham LL13 9UF (GB)**
• **KALINICHEV, Mikhail**
**Wrexham Industrial Estate**
**Wrexham LL13 9UF (GB)**

(74) Representative: **MacLean, Martin Robert**
**Mathys & Squire**
**The Shard**
**32 London Bridge Street**
**London SE1 9SG (GB)**

(56) References cited:
**WO-A2-2006/078588**

• **HEATHER M. MACKENZIE ET AL: "Peri-Incisional Botulinum Toxin for Chronic Postcraniotomy Headache After Traumatic Brain Injury: A Case Series", PM&R, vol. 7, no. 7, 1 July 2015 (2015-07-01), AMSTERDAM, NL, pages 785 - 788, XP055542506, ISSN: 1934-1482, DOI: 10.1016/j.pmrj.2015.02.015**
• **SHILPADEVI PATIL ET AL: "Botulinum Toxin: Pharmacology and Therapeutic Roles in Pain States", CURRENT PAIN AND HEADACHE REPORTS, vol. 20, no. 3, 15 February 2016 (2016-02-15), US, XP055542572, ISSN: 1531-3433, DOI: 10.1007/s11916-016-0545-0**
• **JIHYE PARK ET AL: "Botulinum Toxin for Central Neuropathic Pain", TOXINS, vol. 10, no. 6, 1 June 2018 (2018-06-01), pages 224, XP055542241, DOI: 10.3390/toxins10060224**
• **LIPPERT M.: "Botulinum toxin in the treatment of post-traumatic headache - case study", NEUROLOGIA I NEUROCHIRURGIA POLSKA, vol. 46, no. 6, 1 January 2012 (2012-01-01), PL, pages 591 - 594, XP055268207, ISSN: 0028-3843, DOI: 10.5114/ninp.2012.32109**

**Description**

The present invention is defined by the appended claims.

The present invention relates generally to treating the symptoms of traumatic brain injury (TBI), and more particularly, to a method of treating such symptoms using botulinum neurotoxin. The invention also relates to a composition comprising botulinum toxin useful in treating a symptom of TBI. The invention further relates to a computer system programmed to receive information related to a subject's response to administration of botulinum neurotoxin), store that response in a database, and transmit the response to a medical practitioner. The invention additionally relates to a non-transitory computer-readable storage medium storing instructions that, when executed by a computer system, causes the computer system to perform the aforementioned steps.

According to the U.S. Centers for Disease Control and Prevention (CDC), TBI afflicts approximately 1.7 million people in the United States annually and contributes to about 30% of all deaths from injury.

TBI is defined by the CDC as a disruption in the normal function of the brain that can be caused by a bump, a blow to the head, a fall, a vehicle crash, or penetrating head injury. The risk of TBI increases with age. Symptoms of TBI include temporary loss of consciousness, headache, confusion, sensitivity to light, sensitivity to noise, nausea, vomiting, lack of motor coordination, impaired thinking, loss of memory, impairment of motion, dizziness, light headedness, difficulty balancing, blurred vision or tired eyes, ringing in the ears, bad taste in the mouth, fatigue or lethargy, personality changes, changes in sleep patterns, abnormality in learning and memory, impulsivity, emotional reactivity, mood swings, depression, and emotional blunting.

Typically, TBI is diagnosed following a computer tomography (CT) scan which detects brain tissue damage or intracranial lesions. However, various blood proteins have been linked to the presence of symptoms of TBI, including ubiquitin carboxy-terminal hydrolase L1 (UCH-L1), glial fibrillary acidic protein (GFAP) and calcitonin gene related peptide (CGRP). See Lewis et al., Acad Emerg Med, 24(6) 710-720 (2017) and Bree and Levy, Cephalalgia, 38: 246-258 (2018). Levels of such proteins in the blood of an injured subject may predict which subjects are more likely to develop the symptoms of TBI.

Subjects suffering from the symptoms of TBI are typically treated using oral medications. These medications often must be taken on a daily basis. As such, there is a need for a regimen that allows for less frequent administration.

In addition, headaches are one of the most common symptoms of mild TBI and subjects having headaches often overuse abortive medications and thus develop medication overuse headache. As such, there is also a need for a treatment option that does not rely on the use of abortive medications.

Botulinum neurotoxin is known for use in treating the muscle hyperactivity symptoms of TBI, such as spasticity. In contrast to oral medications, one administration of botulinum neurotoxin is known to last for up to 12 weeks. MacKenzie et al (PM R. 2015 Jul;7(7):785-788) describes a case of peri-incisional botulinum toxin for chronic postcraniotomy headache after traumatic brain injury. Patil et al (Curr Pain Headache Rep. 2016 Mar;20(3):15) describes pharmacology and therapeutic roles of botulinum toxin in pain states. Park and Chung (Toxins. 2018 Jun 1;10(6):224) describes botulinum toxin for central neuropathic pain. WO2006078588A2 describes methods for treating headache. Lippert-Grüner (Neurol Neurochir Pol. 2012 Nov-Dec;46(6):591-4) describes a case of botulinum toxin in the treatment of post-traumatic headache.

Botulinum neurotoxin is produced by *Clostridium botulinum* in the form of a large protein complex comprising botulinum neurotoxin itself complexed to a number of accessory proteins. There are at present seven different classes of botulinum neurotoxin, namely: botulinum neurotoxin serotypes A, A1, A2, A3, A4, B, C, C1, D, E, F and G, all of which share similar structures and modes of action. Different BoNT serotypes can be distinguished based on inactivation by specific neutralizing anti-sera, with such classification by serotype correlating with percentage sequence identity at the amino acid level. Botulinum neurotoxin proteins of a given serotype are further divided into different subtypes on the basis of amino acid percentage sequence identity.

Botulinum neurotoxin type A is commercially available from, e.g., Ipsen (DYSPORT®, Ipsen Limited, Slough, UK) and Allergan (BOTOX®, Allergan Inc., Irvine, CA, USA) whereas botulinum toxin type B is sold by Elan (Myobloc®/Neurobloc®, Solstice Neurosciences Inc., San Diego, CA, USA).

It is believed that botulinum neurotoxin acts by suppressing the release of nociceptive agents and inflammatory agents, such as substance P, glutamate, epinephrine, norepinephrine, and calcitonin gene related peptide as well as by inhibition of voltage gated sodium channels.

The present invention is predicated on the surprising finding that a botulinum neurotoxin may be used to treat TBI-induced symptoms as claimed.

The invention is defined by the claims. In one aspect, there is provided a composition for use in a method of treating a traumatic brain injury-induced symptom in a patient suffering a traumatic brain injury (TBI), the composition comprising a therapeutically-effective amount of a botulinum neurotoxin (BoNT), wherein the composition is administered within 72 hours following injury, and/or wherein the composition is administered up to 4 days pre-injury, wherein the TBI-induced symptom is TBI-induced pain and/or TBI-induced light sensitivity.

The term "a symptom of traumatic brain injury" may be used synonymously with the term "TBI-induced symptom".

The inventors have devised a number of administration strategies providing unexpected technical effects:

- When a botulinum neurotoxin (e.g. Dysport) is administered shortly after TBI (e.g. up to 24 to 72h after the TBI), it can treat/inhibit both acute TBI-induced symptoms (e.g. allodynia) and prevent the onset of (e.g. bright-light stress-induced) symptoms (e.g. allodynia), repeatedly, many weeks after the TBI;

- Pretreatment with a botulinum neurotoxin before the TBI (e.g. up to 4 days before injury) blocks both the acute post-TBI phase and chronic (e.g. light stress-induced) symptoms (e.g. allodynia).

Advantageously, the present invention offers a preemptive treatment (e.g. disease-modifying treatment) opportunity with botulinum neurotoxin, which can prevent a series of subsequent events expressed both in an acute phase (e.g. as an acute headache phase) and chronic phase (e.g. chronic sensitization to light).

In one embodiment, the botulinum neurotoxin is administered pre-injury (reference to the term "injury" throughout means TBI) wherein, the botulinum neurotoxin is administered up to 4 days pre-injury.

In one embodiment, the botulinum neurotoxin is administered between 1-3 days pre-injury. For example, the botulinum neurotoxin may be administered 3 days pre-injury.

The botulinum neurotoxin may be administered to a subject before undergoing an activity which is associated with risk of TBI (such as a sport activity and/or military operation). For example, the TBI may be a sports or military related injury.

One or more biomarkers (such as FDA approved blood biomarkers for identifying "high risk" patient population after the TBI) may be employed in a pre-screening step to identify a subject suitable for receiving treatment with botulinum neurotoxin.

The treatment (of symptoms) may effected at a site that is peripheral to the site of administration; preferably wherein the administration is local administration to the subject's head.

In one embodiment, the method treats at least one symptom that affects a region of the subject's body that is distal to the head.

Advantageously, the inventors have found that local administration (e.g. additional administration) at said regions peripheral / distal to the head is not necessary, as administration to the head (e.g. the cranial suture) following TBI suffices for treating TBI symptoms at regions as distal to the heads as the limbs. This is particularly advantageous where symptoms effect multiple regions of the body, as a single site of administration can be employed, while providing whole-body alleviation of symptoms.

The present inventors have found that a botulinum neurotoxin leads to suppression of a particularly uncomfortable (e.g. associated with a significant decrease in the quality of life) symptom of TBI, namely TBI-induced allodynia (an example of a chronic sensitization). Thus, the present inventors have identified an important subpopulation (or subgroup) of patients which can benefit from treatment with a botulinum neurotoxin.

One aspect of the invention is predicated on the surprising finding that administration of a botulinum neurotoxin treats allodynia resulting from TBI (e.g. TBI-induced allodynia).

Allodynia means "other pain". It is a pain that results from a stimulus that is not normally painful. By way of example, a subject suffering from bad sunburn may experience intense pain even to light touch. In more detail, sun exposure can overly sensitize the skin such that wearing a shirt or taking a shower can be very painful. Thus, a sufferer of tactile allodynia (aka static tactile allodynia or mechanical allodynia) may experience pain to touch, such as with resting one's head on a pillow, or with wearing a hat, earrings, or necklace. Similarly, a sufferer of dynamic allodynia may experience pain from lightly brushing one's hair, or from shaving one's face. Allodynia is a condition that is distinct from "referred" pain (also known as reflective pain), although it can occur outside the area stimulated. It is also distinct from hyperalgesia, which is a pain stimulus that is more painful than usual. Indeed, as mentioned above, allodynia is by it very definition "pain due to a stimulus that does not usually provoke pain", as opposed to hyperalgesia (increased pain from a stimulus that does usually provoke pain).

Allodynia is distinct from "referred" pain and hyperalgesia not only at the symptomatic level, but also at the molecular / cellular level. It is known that peripheral sensitisation and maladaptive central changes contribute to the generation and maintenance of reactions to a sensory modality (e.g. touch, pressure, pinprick, cold, and heat), with separate mechanisms in different subtypes of allodynia and hyperalgesia. The underlying mechanism of "cutaneous allodynia" in migraine has been reported to be due to sensitization of specific set of neurons, namely second-order neurons in the trigeminal nucleus caudalis (TNC).

A difference between allodynia and other types of pain is demonstrated by the fact that certain analgesics are not efficacious for the treatment of allodynia. For example, it has been reported that allodynia shows poor response to the triptans (a class of drug which act as agonists for serotonin 5-HT1B and 5-HT1D receptors at blood vessels and nerve endings in the brain), suggesting that allodynia is not (or is poorly) associated with said receptors, such that they are poor candidate targets for an allodynia treatment.

Opioid management of allodynia is common, and reasonably efficacious. However, the dose range required to attenuate allodynia produces unacceptable cognitive and gastrointestinal side effects, and there exist many patient-types for which opioids are contraindicated. In addition, the now widely acknowledged problem of opioid-dependence casts serious doubts over the future use (especially the long-term use) of this class of drug as a suitable analgesic of choice.

Accordingly, the present invention addresses an increasing need for alternative anti-allodynia therapies/ therapeutics. In particular, the invention addresses a need for alternative analgesics effective in TBI-induced pain (e.g. allodynia).

In a key advantage of the invention, the present invention provides an advantageous re-purposing of a botulinum neurotoxin, providing alternative treatment to addictive analgesics such as opioids (e.g. morphine). This is particularly advantageous for treating patients for whom another (different) pain medicine is contraindicated - by way of example, all triptans are contraindicated in patients with cardiovascular diseases (coronary spasms, symptomatic coronary artery disease, after a heart attack or stroke, uncontrolled hypertension, Raynaud's disease, peripheral artery disease). Most triptans are also contraindicated during pregnancy and breastfeeding and for patients younger than 18. There exist numerous contraindications to opioids (morphine), such as (simply by way of example) systemic mastocytosis, untreated decreased level of thyroid hormones, and decreased function of the adrenal gland.

Botulinum neurotoxin is a non-cytotoxic protease. In contrast to a cytotoxic protease (e.g. ricin, diphtheria toxin, pseudomonas exotoxin), which acts by killing its natural target cell, a non-cytotoxic protease acts by transiently incapacitating the cellular function of its natural target cell. Importantly, a non-cytotoxic protease does not kill the natural target cell upon which it acts. Some of the best known examples of non-cytotoxic proteases include clostridial neurotoxins (e.g. botulinum neurotoxin, which is marketed under names such as Dysport™ Neurobloc™, and Botox™), IgA proteases (see, for example, WO99/032272), and antarease proteases (see, for example, WO2011/022357). In more detail, non-cytotoxic proteases act by proteolytically-cleaving and thus inactivating intracellular transport proteins known as SNARE proteins (e.g. SNAP-25, VAMP, or Syntaxin) - see Gerald K (2002) "Cell and Molecular Biology" (4th edition) John Wiley & Sons, Inc. The acronym SNARE derives from the term Soluble NSF Attachment Receptor, where NSF means N-ethylmaleimide-Sensitive Factor. SNARE proteins are essential components of the vesicular secretion process in eukaryotic cells. Thus, non-cytotoxic proteases act by suppressing cellular secretion. This class of protein includes re-targeted non-cytotoxic proteins in which the natural binding ability of the protein has been modified by the introduction of a binding ligand (also known as a Targeting Moiety), thereby conferring new target cell binding properties on the modified protein. Applicant has pioneered the technology relating to the re-targeting of non-cytotoxic proteases, which dates back to the 1990s (see, for example, WO 94/21300, WO 96/33273 and WO 98/07864). Said re-targeted proteins are referred to (throughout the literature and scientific community) as Targeted Secretion Inhibitors (TSIs) - reference to TSIs includes structural equivalents such as those described in WO 2011/018665.

Examples of suitable botulinum neurotoxins include BoNT/A, BoNT/B, BoNT/C$_1$, BoNT/D, BoNT/E, BoNT/F, BoNT/G, and sub-types thereof. In a preferable embodiment, the botulinum neurotoxin is BoNT/A.

Reference to botulinum neurotoxin embraces serotype hybrids (such as BoNT structural domain serotype hybrids), and modified neurotoxins (such as TSIs), and multi-domain neurotoxins (e.g. multi L-chain BoNTs).

In a preferred embodiment, botulinum neurotoxin of the present invention effects the same sequential intoxication steps that a BoNT/A (e.g. BoNT/A$_1$) is able to demonstrate, namely binding to nerve cells (e.g. via polysialoganglioside receptor) of the presynaptic muscular junction, leading to endosomal release of the protease into the cytosol, and resulting in SNAP-25 cleavage within the cytosol.

By way of example, typical protease (reference) sequences include:

| | |
|---|---|
| .Botulinum type A neurotoxin | - amino acid residues (1-448) |
| Botulinum type B neurotoxin | - amino acid residues (1-440) |
| Botulinum type C neurotoxin | - amino acid residues (1-441) |
| Botulinum type D neurotoxin | - amino acid residues (1-445) |
| Botulinum type E neurotoxin | - amino acid residues (1-422) |
| Botulinum type F neurotoxin | - amino acid residues (1-439) |
| Botulinum type G neurotoxin | - amino acid residues (1-441) |

The above-identified reference sequence should be considered a guide as slight variations may occur according to sub-serotypes. By way of example, US 2007/0166332 cites slightly different clostridial sequences:

| Botulinum type A neurotoxin | - amino acid residues (M1-K448) |
| Botulinum type B neurotoxin | - amino acid residues (M1-K441) |
| Botulinum type C neurotoxin | - amino acid residues (M1-K449) |
| Botulinum type D neurotoxin | - amino acid residues (M1-R445) |
| Botulinum type E neurotoxin | - amino acid residues (M1-R422) |
| Botulinum type F neurotoxin | - amino acid residues (M1-K439) |
| Botulinum type G neurotoxin | - amino acid residues (M1-K446) |

A variety of clostridial toxin fragments comprising the light chain can be useful in aspects of the present invention with the proviso that these light chain fragments can specifically target the core components of the neurotransmitter release apparatus and thus participate in executing the overall cellular mechanism whereby a clostridial toxin proteolytically cleaves a substrate. The light chains of clostridial toxins are approximately 420-460 amino acids in length and comprise an enzymatic domain. Research has shown that the entire length of a clostridial toxin light chain is not necessary for the enzymatic activity of the enzymatic domain. As a non-limiting example, the first eight amino acids of the BoNT/A light chain are not required for enzymatic activity. As another non-limiting example, the first eight amino acids of the TeNT light chain are not required for enzymatic activity. Likewise, the carboxyl-terminus of the light chain is not necessary for activity. As a non-limiting example, the last 32 amino acids of the BoNT/A light chain (residues 417-448) are not required for enzymatic activity. As another non-limiting example, the last 31 amino acids of the TeNT light chain (residues 427-457) are not required for enzymatic activity. Thus, aspects of this embodiment can include clostridial toxin light chains comprising an enzymatic domain having a length of, for example, at least 350 amino acids, at least 375 amino acids, at least 400 amino acids, at least 425 amino acids and at least 450 amino acids. Other aspects of this embodiment can include clostridial toxin light chains comprising an enzymatic domain having a length of, for example, at most 350 amino acids, at most 375 amino acids, at most 400 amino acids, at most 425 amino acids and at most 450 amino acids.

In one embodiment, the botulinum neurotoxin cleaves a non-neuronal SNARE protein such as a SNAP-23 protein. In one embodiment, the botulinum neurotoxin is a modified botulinum toxin L-chain capable of cleaving SNAP-23. An example of such a modified L-chain is described by Chen and Barbieri, PNAS, vol. 106, no. 23, p9180-9184, 2009.

In one embodiment, the botulinum neurotoxin is a BoNT/A, BoNT/C or BoNT/E protease, and the preferred SNARE motif is a SNAP (e.g. SNAP 25) motif. In another embodiment, the non-cytotoxic protease is a BoNT/B, BoNT/D, BoNT/F or BoNT/G, and the preferred SNARE motif is a VAMP motif. In another embodiment, the botulinum neurotoxin is a BoNT/C1 protease, and the preferred SNARE motif is a syntaxin motif.

The polypeptides of the present invention, especially the protease component thereof, may be PEGylated - this may help to increase stability, for example duration of action of the protease component. PEGylation is particularly preferred when the protease comprises a BoNT/A, B or C1 protease. PEGylation preferably includes the addition of PEG to the N-terminus of the protease component. By way of example, the N-terminus of a protease may be extended with one or more amino acid (e.g. cysteine) residues, which may be the same or different. One or more of said amino acid residues may have its own PEG molecule attached (e.g. covalently attached) thereto. An example of this technology is described in WO2007/104567.

The botulinum neurotoxin is preferably a BoNT/A, BoNT/C$_1$ or BoNT/E protease, and the preferred SNARE motif is a SNAP (e.g. SNAP 25) motif.

Alternatively, the botulinum neurotoxin may be a BoNT/B, BoNT/D, BoNT/F or BoNT/G or tetanus neurotoxin (TeNT) protease, and the preferred SNARE motif is a VAMP motif. Alternatively, the botulinum neurotoxin is a BoNT/C$_1$ protease, and the preferred SNARE motif is a syntaxin motif.

In use, the botulinum neurotoxin of the present invention (eg. the L-chain of a clostridial neurotoxin, or equivalent SNARE-cleaving protease) is typically delivered to its target location (i.e. the cytosol of the natural target cell of a clostridial neurotoxin) by cooperation/ coordination with clostridial neurotoxin scaffold components, namely a Targeting Moiety (eg. the H$_C$ domain of a clostridial neurotoxin, or equivalent) and a Translocation Domain (eg. the H$_N$ domain of a clostridial neurotoxin, or equivalent). These scaffold components are well known to a person of skill in the art.

By way of example, typical translocation (reference) sequences include:

| Botulinum type A neurotoxin | - amino acid residues (449-871) |
| Botulinum type B neurotoxin | - amino acid residues (441-858) |
| Botulinum type C neurotoxin | - amino acid residues (442-866) |
| Botulinum type D neurotoxin | - amino acid residues (446-862) |
| Botulinum type E neurotoxin | - amino acid residues (423-845) |
| Botulinum type F neurotoxin | - amino acid residues (440-864) |

(continued)

| Botulinum type G neurotoxin | - amino acid residues (442-863) |

The above-identified reference sequence should be considered a guide as slight variations may occur according to sub-serotypes. By way of example, US 2007/0166332 cites slightly different clostridial sequences:

| | |
|---|---|
| Botulinum type A neurotoxin | - amino acid residues (A449-K871) |
| Botulinum type B neurotoxin | - amino acid residues (A442-S858) |
| Botulinum type C neurotoxin | - amino acid residues (T450-N866) |
| Botulinum type D neurotoxin | - amino acid residues (D446-N862) |
| Botulinum type E neurotoxin | - amino acid residues (K423-K845) |
| Botulinum type F neurotoxin | - amino acid residues (A440-K864) |
| Botulinum type G neurotoxin | - amino acid residues (S447-S863) |

The present invention also embraces variant translocation domains, so long as the variant domains still demonstrate the requisite translocation activity. By way of example, a variant may have at least 70%, preferably at least 80%, more preferably at least 90%, and most preferably at least 95% or at least 98% amino acid sequence homology with a reference translocation domain. The term fragment, when used in relation to a translocation domain, means a peptide having at least 20, preferably at least 40, more preferably at least 80, and most preferably at least 100 amino acid residues of the reference translocation domain. In the case of a clostridial translocation domain, the fragment preferably has at least 100, preferably at least 150, more preferably at least 200, and most preferably at least 250 amino acid residues of the reference translocation domain (eg. $H_N$ domain). As with the TM 'fragment' component (discussed above), translocation 'fragments' of the present invention embrace fragments of variant translocation domains based on the reference sequences.

It is well documented that certain domains of bacterial toxin molecules are capable of forming such pores. It is also known that certain translocation domains of virally expressed membrane fusion proteins are capable of forming such pores. Such domains may be employed in the present invention.

Other examples include the translocation domain of diphtheria toxin, the translocation domain of *Pseudomonas* exotoxin type A, the translocation domains of anthrax toxin, influenza virus haemagglutinin, Semliki Forest virus fusogenic protein, Vesicular Stomatitis virus glycoprotein G, SER virus F protein, and Foamy virus envelope glycoprotein.

In the context of the present invention, a variety of clostridial toxin $H_N$ regions comprising a translocation domain can be useful in aspects of the present invention with the proviso that these active fragments can facilitate the release of a non-cytotoxic protease (e.g. a clostridial L-chain) from intracellular vesicles into the cytoplasm of the target cell and thus participate in executing the overall cellular mechanism whereby a clostridial toxin proteolytically cleaves a substrate. The $H_N$ regions from the heavy chains of Clostridial toxins are approximately 410-430 amino acids in length and comprise a translocation domain. Research has shown that the entire length of a $H_N$ region from a Clostridial toxin heavy chain is not necessary for the translocating activity of the translocation domain. Thus, aspects of this embodiment can include clostridial toxin $H_N$ regions comprising a translocation domain having a length of, for example, at least 350 amino acids, at least 375 amino acids, at least 400 amino acids and at least 425 amino acids. Other aspects of this embodiment can include clostridial toxin $H_N$ regions comprising translocation domain having a length of, for example, at most 350 amino acids, at most 375 amino acids, at most 400 amino acids and at most 425 amino acids.

For further details on the genetic basis of toxin production in *Clostridium botulinum* and *C. tetani*, we refer to Henderson et al (1997) in The Clostridia: Molecular Biology and Pathogenesis, Academic press. The term $H_N$ embraces naturally-occurring neurotoxin $H_N$ portions, and modified $H_N$ portions having amino acid sequences that do not occur in nature and/ or synthetic amino acid residues, so long as the modified $H_N$ portions still demonstrate the above-mentioned translocation function.

Alternatively, the Translocation Domain may be of a non-clostridial origin. Examples of non-clostridial (reference) Translocation Domain origins include, but not be restricted to, the translocation domain of diphtheria toxin [O'Keefe et al., Proc. Natl. Acad. Sci. USA (1992) 89, 6202-6206; Silverman et al., J. Biol. Chem. (1993) 269, 22524-22532; and London, E. (1992) Biochem. Biophys. Acta., 1112, pp.25-51], the translocation domain of Pseudomonas exotoxin type A [Prior et al. Biochemistry (1992) 31, 3555-3559], the translocation domains of anthrax toxin [Blanke et al. Proc. Natl. Acad. Sci. USA (1996) 93, 8437-8442], a variety of fusogenic or hydrophobic peptides of translocating function [Plank et al. J. Biol. Chem. (1994) 269, 12918-12924; and Wagner et al (1992) PNAS, 89, pp.7934-7938], and amphiphilic peptides [Murata et al (1992) Biochem., 31, pp.1986-1992]. The Translocation Domain may mirror the Translocation Domain present in a naturally-occurring protein, or may include amino acid variations so long as the variations do not destroy the translocating ability of the Translocation Domain.

Particular examples of viral (reference) Translocation Domains suitable for use in the present invention include certain translocating domains of virally expressed membrane fusion proteins. For example, Wagner et al. (1992) and Murata et al. (1992) describe the translocation (i.e. membrane fusion and vesiculation) function of a number of fusogenic and amphiphilic peptides derived from the N-terminal region of influenza virus haemagglutinin. Other virally expressed membrane fusion proteins known to have the desired translocating activity are a translocating domain of a fusogenic peptide of Semliki Forest Virus (SFV), a translocating domain of vesicular stomatitis virus (VSV) glycoprotein G, a translocating domain of SER virus F protein and a translocating domain of Foamy virus envelope glycoprotein. Virally encoded Aspike proteins have particular application in the context of the present invention, for example, the E1 protein of SFV and the G protein of the G protein of VSV.

Use of the (reference) Translocation Domains includes use of sequence variants thereof. A variant may comprise one or more conservative nucleic acid substitutions and/ or nucleic acid deletions or insertions, with the proviso that the variant possesses the requisite translocating function. A variant may also comprise one or more amino acid substitutions and/ or amino acid deletions or insertions, so long as the variant possesses the requisite translocating function.

The polypeptides of the present invention may further comprise a translocation facilitating domain. Said domain facilitates delivery of the botulinum neurotoxin into the cytosol of the target cell and are described, for example, in WO 08/008803 and WO 08/008805.

By way of example, suitable translocation facilitating domains include an enveloped virus fusogenic peptide domain, for example, suitable fusogenic peptide domains include influenzavirus fusogenic peptide domain (eg. influenza A virus fusogenic peptide domain of 23 amino acids), alphavirus fusogenic peptide domain (eg. Semliki Forest virus fusogenic peptide domain of 26 amino acids), vesiculovirus fusogenic peptide domain (eg. vesicular stomatitis virus fusogenic peptide domain of 21 amino acids), respirovirus fusogenic peptide domain (eg. Sendai virus fusogenic peptide domain of 25 amino acids), morbilivirus fusogenic peptide domain (eg. Canine distemper virus fusogenic peptide domain of 25 amino acids), avulavirus fusogenic peptide domain (eg. Newcastle disease virus fusogenic peptide domain of 25 amino acids), henipavirus fusogenic peptide domain (eg. Hendra virus fusogenic peptide domain of 25 amino acids), metapneumovirus fusogenic peptide domain (eg. Human metapneumovirus fusogenic peptide domain of 25 amino acids) or spumavirus fusogenic peptide domain such as simian foamy virus fusogenic peptide domain; or fragments or variants thereof.

By way of further example, a translocation facilitating domain may comprise a Clostridial toxin $H_{CN}$ domain or a fragment or variant thereof. In more detail, a Clostridial toxin $H_{CN}$ translocation facilitating domain may have a length of at least 200 amino acids, at least 225 amino acids, at least 250 amino acids, at least 275 amino acids. In this regard, a *Clostridial* toxin $H_{CN}$ translocation facilitating domain preferably has a length of at most 200 amino acids, at most 225 amino acids, at most 250 amino acids, or at most 275 amino acids. Specific (reference) examples include:

| | |
|---|---|
| Botulinum type A neurotoxin | - amino acid residues (872-1110) |
| Botulinum type B neurotoxin | - amino acid residues (859-1097) |
| Botulinum type C neurotoxin | - amino acid residues (867-1111) |
| Botulinum type D neurotoxin | - amino acid residues (863-1098) |
| Botulinum type E neurotoxin | - amino acid residues (846-1085) |
| Botulinum type F neurotoxin | - amino acid residues (865-1105) |
| Botulinum type G neurotoxin | - amino acid residues (864-1105) |
| Tetanus neurotoxin | - amino acid residues (880-1127) |

The above sequence positions may vary a little according to serotype/ sub-type, and further examples of suitable (reference) Clostridial toxin $H_{CN}$ domains include:

| | |
|---|---|
| Botulinum type A neurotoxin | - amino acid residues (874-1110) |
| Botulinum type B neurotoxin | - amino acid residues (861-1097) |
| Botulinum type C neurotoxin | - amino acid residues (869-1111) |
| Botulinum type D neurotoxin | - amino acid residues (865-1098) |
| Botulinum type E neurotoxin | - amino acid residues (848-1085) |
| Botulinum type F neurotoxin | - amino acid residues (867-1105) |
| Botulinum type G neurotoxin | - amino acid residues (866-1105) |
| Tetanus neurotoxin | - amino acid residues (882-1127) |

Any of the above-described facilitating domains may be combined with any of the previously described translocation domain peptides that are suitable for use in the present invention. Thus, by way of example, a non-clostridial facilitating domain may be combined with non-clostridial translocation domain peptide or with clostridial translocation domain peptide. Alternatively, a Clostridial toxin $H_{CN}$ translocation facilitating domain may be combined with a non-clostridial translocation domain peptide. Alternatively, a Clostridial toxin $H_{CN}$ facilitating domain may be combined or with a clostridial translocation domain peptide, examples of which include:

| | |
|---|---|
| Botulinum type A neurotoxin | - amino acid residues (449-1110) |
| Botulinum type B neurotoxin | - amino acid residues (442-1097) |
| Botulinum type C neurotoxin | - amino acid residues (450-1111) |
| Botulinum type D neurotoxin | - amino acid residues (446-1098) |
| Botulinum type E neurotoxin | - amino acid residues (423-1085) |
| Botulinum type F neurotoxin | - amino acid residues (440-1105) |
| Botulinum type G neurotoxin | - amino acid residues (447-1105) |
| Tetanus neurotoxin | - amino acid residues (458-1127) |

By way of example, typical Targeting Moiety (reference) sequences include:

| | |
|---|---|
| Botulinum type A neurotoxin | - amino acid residues (Y1111-L1296) |
| Botulinum type B neurotoxin | - amino acid residues (Y1098-E1291) |
| Botulinum type C neurotoxin | - amino acid residues (Y1112-E 1291) |
| Botulinum type D neurotoxin | - amino acid residues (Y1099-E1276) |
| Botulinum type E neurotoxin | - amino acid residues (Y1086-K1252) |
| Botulinum type F neurotoxin | - amino acid residues (Y1106-E1274) |
| Botulinum type G neurotoxin | - amino acid residues (Y1106-E1297) |
| Tetanus neurotoxin | - amino acid residues (Y1128-D1315). |

The botulinum neurotoxin may, for example, have at least about 50%, 60%, 70%, 80%, 90%, 95%, 97%, 98%, or 99% sequence identity to the amino acid sequence of a botulinum neurotoxin of types A; or A1; or A2; or A3; or A4; or B; or C; or C1; or D; or E; or F; or G. In one embodiment, the botulinum neurotoxin is DYSPORT® (e.g. BoNT/A).

In the present invention, the botulinum toxin-producing strain is preferably *Clostridium botulinum,* but is not limited thereto, and it will be apparent to those skilled in the art that any strain capable of producing a botulinum toxin may be used in the present invention. As used herein, the term "botulinum toxin" is meant to include not only a neurotoxin produced biologically, but also any modified, recombinant, hybrid, fusion, and chimeric botulinum toxins. A modified or recombinant botulinum toxin may, for example, contain modifications as compared to wild-type botulinum neurotoxins (*e.g.* in their amino acid sequence) so as to have similar or better properties than the wild-type botulinum neurotoxins. In addition, the term "botulinum toxin" as used herein is meant to include any and all known botulinum toxin serotypes, including serotypes A, A1, A2, A3, A4, B, C, C1, D, E, F and G, as well as botulinum toxin complexes (e.g., 300, 600 and 900 kDa complexes), and high purity botulinum neurotoxins (botulinum neurotoxins that are free from complexes with other proteins), which are all useful in the practice of the present invention. For additional information regarding the properties of the various botulinum toxins, reference is made to Simpson D.M. et al, "Assessment: Botulinum neurotoxin for the treatment of spasticity (an evidence-based review): Report of the Therapeutics and Technology Assessment Subcommittee of the American Academy of Neurology," Neurology 6;70(19), 1691-1698 (2008).

In one embodiment, the botulinum neurotoxin is of types A, A1, A2, A3, A4, B, C, C1, D, E, F or G. In another embodiment, the botulinum neurotoxin is a protein containing modifications as compared to wild-type botulinum neurotoxins. In one embodiment, the botulinum neurotoxin is a protein having an amino acid sequence that has at least 50%, 60%, 70%, 80%, 90%, 95%, 97%, 98%, or 99% sequence identity to the amino acid sequence of a botulinum neurotoxin of types A, A1, A2, A3, A4, B, C, C1, D, E, F or G. See WO2015004461, WO2015166242, WO2016110662, WO2006114308, WO2013180799, and WO2017191315.

Botulinum toxin may be obtained commercially by establishing and growing cultures of C. *botulinum* in a fermenter, and harvesting and purifying the fermented mixture in accordance with known techniques. The "A" form of botulinum toxin is currently available commercially from several sources, for example, from Ipsen Biopharmaceuticals Limited under the tradename DYSPORT®, from Merz Pharma under the tradename XEOMIN®, from Medytox Inc. under the tradename CORETOX®, and from Allergan Inc. under the tradename BOTOX®. In one embodiment of the present invention, the botulinum neurotoxin is one of these commercially-available forms.

In a particular embodiment, the botulinum neurotoxin is DYSPORT®. DYSPORT® is an injectable form of botulinum toxin type A (BoNT-A), which is isolated and purified from *Clostridium* bacteria producing BoNT-A. It is supplied as a lyophilized powder. DYSPORT® has approved therapeutic indications in the United States for the treatment of adults with Cervical Dystonia (CD), the treatment of Upper Limb Spasticity (ULS) in adult subjects, and the treatment of lower limb spasticity in children to improve tone and spasticity. The medicine was first registered in the United Kingdom in 1990 for other uses and is licensed in more than 80 countries in eight different indications, with over 1,300 peer-reviewed publications.

In another embodiment, the botulinum toxin used is type B toxin, e.g., as marketed under the trade names MYOBLOC® and NEUROBLOC®.

In one embodiment, the botulinum neurotoxin is naturally-occurring. In another embodiment, the botulinum neurotoxin is produced via recombinant means.

In one embodiment, the botulinum neurotoxin is of type A, also known as botulinum neurotoxin A. In a particular embodiment, the botulinum neurotoxin is DYSPORT®.

It is believed that botulinum neurotoxin acts by suppressing the release of nociceptive agents and inflammatory agents, such as substance P, glutamate, and calcitonin gene related peptide. Cui et al. Pain 107: 125-133 (2004), Marino et al. Pain, 155: 674-684 (2014). Cernuda-Morollon et al. Pain 156: 820-824 (2015), and Lee et al. Anesth Analg, 112: 228-235 (2011).

In one embodiment, the amount of botulinum neurotoxin administered is based on the severity of the TBI, the severity of the symptom of TBI, the weight of the subject, whether the subject has previously experienced TBI, and/or the amount of time that has elapsed since the injury.

In one embodiment, botulinum neurotoxin is administered to a subject diagnosed to be in need thereof following a trauma-inducing injury. In one embodiment, the botulinum toxin is administered following the immediate aftermath of the injury, such as at least about 1, 2, 3, 4, 5, 6, 7, 8, 9, 10, 12, 14, 16, 18, 20, 24, 28, 32, 36, 40, 44, 48, 52, 56, 60, 64, 68, or 72 hours following the injury. For example, the botulinum toxin may be administered 2 to 72 hours following the immediate aftermath of the injury.

TBI is preferably treated immediately following injury. However, such immediate treatment is not always possible or practical. As such, there is a need for an effective TBI treatment in cases where the subjects are unable to receive immediate treatment. To this end, it has been surprisingly discovered that DYSPORT®, initially administered 2 to 72 hours following trauma-inducing injury, is effective in treating the symptoms of TBI.

In one embodiment, the subject's response to treatment with botulinum neurotoxin is measured following an administration of botulinum neurotoxin. Thereafter, a dosing regimen for botulinum neurotoxin may be determined based on the subject's response. The subject's response may be recorded, for example, onto a computer device.

In one embodiment, the method involves determining whether the subject has or is likely to develop a symptom of TBI. Such may, for example, be determined by measuring the level of a biomarker in the subject (preferably in an isolated blood sample of the subject), the level being indicative of whether the subject has or is likely to develop such symptoms. The biomarker may, for example, be ubiquitin carboxy-terminal hydrolase L1 (UCH-L1), glial fibrillary acidic protein (GFAP), calcitonin gene related peptide, TNF-$\alpha$, or NF-L. Preferably, the biomarker is one or more selected from UCH-L1 and GFAP. Preferably, the concentration of said one or more biomarker (e.g UCH-L1 and/or GFAP) is increased in a subject that has or is likely to develop a symptom of TBI, i.e increased relative to the concentration of said one or more biomarker in a healthy subject. Suitably, the healthy subject is a non-TBI subject (e.g. a subject that has not received a traumatic brain injury).

The terms "subject", "individual" and "patient" are used interchangeably herein to refer to a mammalian subject. A "subject" as used herein refers to a mammal, preferably a human, in need of treatment of a symptom of TBI. For example, a subject may be a human patient.

The administration of botulinum toxin is typically performed by a medical professional. As used herein, the term "medical professional" includes a clinician, physician, nurse, medical technician, or the like. However, in some embodiments, the subject may self-administer the botulinum toxin.

In methods of the invention, the subject may not have been previously diagnosed as having a TBI. Alternatively, the subject may have been previously diagnosed as having a TBI. The subject may also be one who is suffering from or is at risk of developing a TBI.

The botulinum toxin may be administered by any means known in the art, including parenterally, intra-muscularly, intradermally, and transdermally. In one embodiment, botulinum neurotoxin is administered parenterally, for example subcutaneously or intravenously. In one embodiment, botulinum neurotoxin is administered subcutaneously.

In one embodiment, the administration is local administration to the subject's head.

Administration may, for example, be through a suture of the skull, for example, the sagittal suture, the coronal suture, or the lambdoid sutures. In one embodiment, administration is through the sagittal suture. Administration through a suture of the skull may, for example, be accomplished subcutaneously. For example, administration may be subcutaneous along the lambdoid suture.

The botulinum toxin can be presented as a sterile pyrogen-free aqueous solution or dispersion, or as a sterile powder for reconstitution into a sterile solution or dispersion. If desired, tonicity adjusting agents, such as sodium chloride, glycerol and/or various sugars can be added. Stabilizers may be included if desired. The formulation may be preserved by means of any suitable pharmaceutically acceptable preservative, such as a paraben.

In some embodiments, the botulinum toxin is formulated in unit dosage form, for example, as a sterile solution in a vial, or as a vial or sachet containing a lyophilized powder for reconstituting in a suitable carrier, such as saline, for injection. In one aspect, the botulinum toxin is formulated in a solution containing saline and pasteurized human serum albumin, which stabilizes the toxin. The solution may be sterile filtered, filled into individual vials, and then vacuum dried to give a sterile lyophilized powder. In use, the powder can be reconstituted by the addition of sterile unpreserved normal saline (sodium chloride 0.9% for injection).

The potency of the toxin may be expressed as a multiple of the $LD_{50}$ value. One $LD_{50}$ unit is the equivalent amount of toxin which causes the death of 50% (one-half) of a group of test animals, such as laboratory mice. Alternative methods of determining the potency of the toxin may also be employed, including, for example, any method included in the *European Pharmacopoeia* monograph 01/2005:2113.

As used herein, the term "unit" refers to a unit dose of DYSPORT®, which refers to the median intraperitoneal $LD_{50}$ dose of DYSPORT® in mice. It is noted that the median intraperitoneal $LD_{50}$ dose of DYSPORT® in mice is not necessarily the same as the median intraperitoneal $LD_{50}$ dose of another botulinum neurotoxin-containing product, such as BOTOX®, as different pharmaceutical preparations are produced differently. For example, units of BOTOX®, as described in the art, are not the same as the units of the present invention, which are units of DYSPORT®.

The term "treat" or "treating" as used herein encompasses prophylactic treatment as well as corrective treatment. In a preferable embodiment, the term "treat" or "treating" as used herein means corrective treatment. In some embodiments the term "treat" or "treating" refers to a symptom of a condition described herein.

Therefore, a non-cytotoxic protease may be administered to a subject in a therapeutically effective amount or a prophylactically effective amount.

A "therapeutically effective amount" is any amount of the botulinum neurotoxin, which when administered alone or in combination to a subject for treatment of a condition described herein is sufficient to effect such treatment of the condition, or symptom thereof.

A "prophylactically effective amount" is any amount of the botulinum neurotoxin that, when administered alone or in combination to a subject inhibits or delays the onset or reoccurrence of a condition described herein (or a symptom thereof). In some embodiments, the prophylactically effective amount prevents the onset or reoccurrence of a condition described herein entirely. "Inhibiting" the onset means either lessening the likelihood of onset of a condition described herein (or symptom thereof), or preventing the onset entirely.

For example, embodiments wherein the botulinum neurotoxin is administered pre-injury, the botulinum neurotoxin suitably be administered in a prophylactically effective amount.

Typically, the therapeutically-effective amount of the botulinum toxin administered to the subject is sufficient to reduce the symptoms of TBI. The amount of the botulinum toxin may depend upon a variety of factors, including the method of administration, the weight of the subject, severity of the subject's condition (e.g., the severity of the subject's TBI or the severity of the subject's symptoms of TBI), the type and potency of the particular toxin, the status of the subject's recovery, the amount of elapsed time following injury, the subject's history with botulinum neurotoxin, whether the subject has previously experienced, the subject's height, the subject's body surface area, the subject's age, and/or the level of certain biomarkers in the blood of the subject. In one embodiment, the dosage is the lowest amount of botulinum neurotoxin which is therapeutically-effective. The administrations may to be repeated as necessary, though subsequent administrations may vary in dosage, administration route, and/or toxin type.

In one embodiment, the botulinum neurotoxin is administered in a single dose (e.g. a single dose may effect the treatment).

In one embodiment, the amount of botulinum neurotoxin administered is between 20-70 units. For example, the amount of botulinum neurotoxin administered may be about 50 units.

In one embodiment, Dysport® is administered per treatment session in an amount of about 30 units per kilogram of the total body weight of the subject (units/kg) or less, for example from about 1 to about 30 units/kg. In other embodiments, it may be administered in an amount of about 10 to about 20 units/kg or about 15 units/kg per session.

For example, a total dosage of 1 to 30 units/kg of the total body weight of the subject may be administered to a subject per treatment session. As such, 20 to 600 units may be administered to a subject having a total body weight of 20 kg and 40 to 1,200 units may be administered to a subject having a total body weight of 40 kg. The total maximum dosage of Dysport® to a subject per session is 1,500 units.

In further embodiments, Dysport® is administered to a subject in an amount of from 250 to 550 units, 350 to 450 units, or about 400 units per treatment session.

In one embodiment, the TBI is mild TBI (mTBI). For example, a traumatic brain injury (TBI) can be classified as "mild" if loss of consciousness and/or confusion and disorientation is shorter than 30 minutes. While MRI and CAT scans are often normal, the individual has cognitive problems such as headache, difficulty thinking, memory problems, attention deficits, mood swings and frustration. Other names of mTBI include concussion, minor head trauma, minor TBI, minor brain injury, and minor head injury.

In one embodiment, the amount of botulinum neurotoxin administered to the subject may be directly proportional to the severity of the TBI. For example, a dosage of from about 1 to about 10 units/kg may be administered per treatment session to a subject having a mild TBI. A dosage of from about 10 to about 20 units/kg may be administered per session to a subject having a moderate TBI. A dosage of from about 20 to about 30 units/kg may be administered per session to a subject having a severe TBI.

One measure of the severity of the TBI is the duration of loss of consciousness (LOC) suffered by the subject following TBI. A mild TBI correlates with a LOC of less than about 20 minutes. Moderate TBI correlates with a LOC of from about 20 minutes to about 6 hours. Severe TBI correlates with a LOC of over about 6 hours. In view of the above, a dosage of from about 1 to about 10 units/kg per treatment session may, for example, be administered to a subject that has experienced an LOC of less than about 20 minutes. A dosage of from about 10 to about 20 units/kg per treatment session may, for example, be administered to a subject that has experienced an LOC of from about 20 minutes to about 6 hours. A dosage of from about 20 to about 30 units/kg per treatment session may be administered to a subject that has experienced an LOC of over about 6 hours.

Another measure of the severity of the TBI is the length of post-traumatic amnesia (PTA). PTA is a state of confusion that occurs after a subject regains consciousness following TBI. During PTA, the subject may be unable to state his/her name, where he/she is, or what time it is. The subject is also unable to store new events in his/her memory. PTA is resolved when the subject regains continuous memory. A mild TBI correlates with a PTA of less than about one hour, as measured after the subject regains consciousness. Moderate TBI correlates with a PTA of from about 1 hour to about 24 hours. Severe TBI correlates with a PTA of over about 24 hours. In view of the above, a dosage of from about 1 to about 10 units/kg per treatment session may, for example, be administered to a subject that has experienced a PTA of less than about one hour. A dosage of from about 10 to about 20 units/kg per session may, for example, be administered to a subject that has experienced a PTA of from about 1 hour to about 24 hours. A dosage of from about 20 to about 30 units/kg per session may be administered to a subject that has experienced a PTA of over about 24 hours.

Yet another measure of the severity of the TBI is the Glasgow Coma Scale (GCS), which measures the conscious state of a person by measuring ocular, verbal, and motor responses. The following scores correlate to the following eye conditions: (1) subject does not open eyes; (2) subject opens eyes in response to pain; (3) subject opens eyes in response to speech; and (4) subject opens eyes spontaneously. The following scores correlate to the following verbal conditions: (1) subject makes no sounds; (2) subject makes incomprehensible sounds; (3) subject utters incoherent words; (4) subject's verbal response is confused; and (5) subject's verbal response is oriented. The following scores correlates to the following motor conditions: (1) subject makes no movements; (2) subject extends muscles in response to pain; (3) subject exhibits abnormal flexion to painful stimuli; (4) subject is capable of withdrawing from painful stimuli; (5) subject moves limb to avoid pain; and (6) subject is capable of obeying commands. The motor response can be measured using any limb. The scores for all three tests are then added. Mild TBI correlates with a total GCS score of 13 or higher. Moderate TBI correlates with a total GCS score of 9 to 12. Severe TBI correlates with a total GCS score of 8 or less. In view of the above, a dosage of from about 1 to about 10 units/kg may, for example, be administered to a subject that has a GCS score of 13 or higher. A dosage of from about 10 to about 20 units/kg per treatment session may, for example, be administered to a subject that has a GCS score of 9 to 12. A total dosage of from about 20 to about 30 units/kg per session may be administered to a subject that has a GCS score of 8 or less.

Subjects suffering from TBI exhibit various symptoms. Symptoms of TBI are defined by the claims.

In one embodiment, the symptom of TBI is a headache, for example, a post-traumatic headache or a migraine headache. Post-traumatic headache may be acute or chronic. In another embodiment, the symptom of TBI is sensitivity to light.

In one embodiment, the amount of botulinum neurotoxin administered to the subject may be directly proportional to the severity of the symptoms of TBI.

In addition, a lower dose of botulinum neurotoxin may be administered if the subject has exhibited adverse effects following previous treatment with botulinum neurotoxin. Examples of adverse effects include: respiratory tract infection, nasopharyngitis, influenza, pharyngitis, cough, pyrexia, bronchitis, rhinitis, varicella, ear infection, gastroenteritis, vomiting, nausea, oropharyngeal pain, pain in extremities, muscular weakness, convulsion, and/or epilepsy. Examples of such a lower dose are from about 1 to about 20 units/kg or from about 1 to about 10 units/kg of the total body weight of the subject per treatment session.

In one embodiment, the subject's response is recorded into a software program that is configured to receive such information. The program may operate on a computer device. Based on the subject's response as measured, the program may determine an appropriate dosing regimen for a botulinum neurotoxin.

Not all subjects that experience TBI develop the symptoms of TBI. As such, it may be important to determine the likelihood a subject suffering TBI may develop such symptoms. This may be accomplished by conducting a computer tomography (CT) scan or magnetic resonance imaging (MRI), which can detect brain tissue damage or intracranial lesions. If such damage or lesions are detected, the subject may be deemed as likely to develop the symptoms of TBI.

In another embodiment, the levels of certain biomarkers in the subject's blood are measured. These levels may be correlative of whether the subject has or is likely to develop one or more symptoms of TBI. Examples of such biomarkers include: ubiquitin carboxy-terminal hydrolase L1 (UCH-L1), glial fibrillary acidic protein (GFAP), neuroin-specific enolase (NSE), calcitonin gene related peptide (CGRP), TNF-$\alpha$, S100$\beta$, spectrin breakdown products (e.g. SBDP125, SBDP145, and SDP150), c-tau, NF-H, and NF-L. For example, levels of such biomarkers above a certain amount may be indicative of the subject having or being likely to develop the symptoms of TBI.

In one embodiment, the dosage is the lowest amount of botulinum neurotoxin which is therapeutically-effective. The administrations are to be repeated as necessary, though subsequent administrations may vary in dosage, administration route, and/or toxin type.

In one embodiment, the method of the present invention involves determining an appropriate dosage amount for botulinum neurotoxin based on an assessment of one or more of the following criteria: the subject's weight, the severity of the TBI, the severity of the symptoms of the TBI, the status of the subject's recovery following TBI, the elapsed period following injury, the subject's history with treatment with botulinum neurotoxin, whether the subject has previously experienced TBI, the subject's height, the subject's body surface area, the level of certain biomarkers in the blood of the subject, and/or the subject's response to a previous treatment with botulinum neurotoxin.

The method may also involve adjusting future doses or the dosing regimen based on the subject's response to a previous treatment.

Depending on the potency of the botulinum toxin and its duration of action, the doses may need to be administered intermittently. Ultimately, however, both the quantity of toxin administered, and the frequency of its administration will be at the discretion of the medical practitioner(s) responsible for the treatment, and should be commensurate with questions of safety and the effects produced by the toxin.

As discussed above, subjects experiencing TBI can be benefited by receiving early botulinum toxin treatment. Accordingly, in some embodiments of the method, a subject experiencing TBI or symptoms of TBI is administered botulinum neurotoxin immediately following an injury or following a diagnosis of TBI or the symptoms of TBI. In some embodiments, a subject experiencing TBI or symptoms of TBI is administered botulinum neurotoxin following the immediate aftermath of the injury, such as at least about 1 hour following the injury. In other embodiments, the subject experiencing TBI or symptoms of TBI is administered botulinum neurotoxin at least about 2 hours, 3 hours, 4 hours, 5 hours, 6 hours, 7 hours, 8 hours, 9 hours, 10 hours, 12 hours, 14 hours, 16 hours, 18 hours, 20 hours, 24 hours, 28 hours, 32 hours, 36 hours, 40 hours, 44 hours, 48 hours, 52 hours, 56 hours, 60 hours, 64 hours, 68 hours, or 72 hours following the injury. In certain embodiments, botulinum neurotoxin is initially administered 2 to 72 hours following injury, 2 to 48 hours following injury, or 2 to 24 hours following injury.

In one embodiment, the amount of units administered to the subject may be directly proportional to the amount of time that has elapsed following injury. As such, higher doses may be desirable if a longer period of time has elapsed. For example, a dose of from about 1 to about 10 units/kg per treatment session may be appropriate immediately following injury (i.e., within the first hour), a dose of from about 10 to about 20 units/kg per session may be appropriate if administered between about 1 hour and about 24 hours following injury, and a dose of from about 20 to about 30 units/kg per session may be appropriate if administered about 24 hours or more following injury.

As noted, it is not always possible to administer botulinum neurotoxin (e.g., DYSPORT®) to a subject immediately following injury. It has surprisingly been found, however, that DYSPORT®, initially administered two or more hours after the trauma-inducing injury may still be effective in treating the symptoms of TBI. Specifically, the inventors of the present invention have discovered that botulinum neurotoxin, in particular DYSPORT®, is effective in treating the symptoms of TBI when administered to subjects at least about 1 hour following injury, and is even effective in treating the symptoms of TBI when administered to subjects at least about 2 hours, 3 hours, 4 hours, 5 hours, 6 hours, 7 hours, 8 hours, 9 hours, 10 hours, 12 hours, 14 hours, 16 hours, 18 hours, 20 hours, 24 hours, 28 hours, 32 hours, 36 hours, 40 hours, 44 hours, 48 hours, 52 hours, 56 hours, 60 hours, 64 hours, 68 hours, or 72 hours following the trauma-inducing injury.

In some embodiments, following treatment with botulinum neurotoxin, the amount or frequency of a subsequent administration or administrations of botulinum neurotoxin may be determined. In one embodiment, the amount of or frequency of such subsequent administrations(s) is determined based, at least in part, on the subject's recovery following a previous treatment with botulinum neurotoxin.

The status of a subject's recovery can be measured using routine methods known to persons of ordinary skill in the art. For example, a subject's recovery status may be tracked on the Rancho Los Amigos scale. The Rancho Los Amigos scale measures response from Level I to Level VIII. Level I indicates that the subject does not respond to sounds, movement, or touch. Level II indicates that the subject exhibits generalized response to stimuli. Level III indicates that the subject exhibits a localized response to stimuli. Level IV indicates that the subject exhibits a confused and agitated response to stimuli. Level V indicates that the subject exhibits a confused and inappropriate response to stimuli. Level VI indicates that the subject exhibits a confused but appropriate response to stimuli (subject is capable of goal-directed behavior but requires external direction). Level VII indicates that the subject exhibits an automatic and appropriate response to stimuli (subject exhibits an appropriate response to stimuli but has a shallow recall of the action(s)). Level VIII indicates that the subject exhibits a purposeful and appropriate response to stimuli. The amount of a dose or dosing frequency may, for example, be increased for a subject exhibiting a Level I or II response, the amount of a dose or dosing frequency may, for example, be maintained for a subject exhibiting a Level III to V response, the amount of a dose or dosing frequency may, for example, be reduced for a subject exhibiting a level subject exhibiting a Level VI or VII response, and further treatment with botulinum neurotoxin may be terminated for a subject exhibiting a Level VIII response.

A subject's response may also be measured using the Physician's Global Assessment (PGA) of response. The PGA measures response on a scale from -4 to +4, with -4 indicating that response is markedly worse, to 0, which indicates no change, to +4 indicating that response has markedly improved. For example, a desired response may be a PGA score following administration of 0.25 or above, 0.5 or above, 1 or above, 2 or above, 3 or above, or 4. As such, the amount of a dose or dosing frequency may, for example, be increased for a subject exhibiting a PGA score of below 0, the amount of a dose or dosing frequency may, for example, be maintained for a subject exhibiting a PGA score of from 0 to 2, the amount of a dose or dosing frequency may, for example, be reduced for a subject exhibiting a level subject exhibiting a PGA score of between 2 and 4, and further treatment with botulinum neurotoxin may be terminated for a subject exhibiting a PGA score of 4 or above.

Another method of measuring a subject's response is the Physician's Global Impressions (PGI) questionnaire.

In some embodiments, the information relating to a subject's response to treatment with botulinum neurotoxin is recorded into a log or diary. In other embodiments, the response information is recorded into or by a software program that operates on a computer device and the response information is stored in a database associated with the computer device. In yet other embodiments, the response information is recorded into or by a software program that operates on a first computer device and the information is stored into a database on the first computer device or a second computer device that is in communication with first computer device.

In some embodiments, the method may additionally comprise administering a subsequent administration of botulinum toxin, wherein the subsequent administration is determined, at least in part, by the response information. In particular embodiments, the method further comprises: reviewing the subject's response information; designing a dosing regimen based, at least in part, on the subject's response information; and administering a subsequent botulinum toxin administration to the subject in accordance with the dosing regimen, wherein the botulinum toxin administration is sufficient to treat or reduce the impaired active movement capacity.

In some embodiments of the invention, the clinical management of the subject's recovery is enhanced by virtue of the fact that information about the subject's recovery is being recorded into a log or diary, or into a software program that is able to receive and communicate information about the subject's recovery.

The software program is capable of running on any suitable computer device. Various information may be received by the software program, including: the types of tests performed, the time that has elapsed since injury, the time that has elapsed since the previous administration, frequency of administration, symptoms relating to TBI, the effect (if any) of the administration, and/or any other comments about the administration and recovery process. The software program receives the information that is associated with the subject's treatment and/or recovery and stores that information into a database. The database can reside on the same computer device used by the practitioner or on a different computer device that is able to communicate with the computer device used by the practitioner.

In some embodiments, the medical practitioner reviews the response information. Using a computer device that is able to communicate with the database, the medical practitioner can access the response information. In one aspect, after reviewing the information, the medical practitioner (e.g., clinician) uses this information to determine an appropriate dosing regimen, or to make or suggest an adjustment to an existing dosing regimen. In another aspect, the medical practitioner uses the response information to determine or vary the botulinum toxin dosing regimen, i.e., the mode, amount, or frequency of botulinum toxin administered to the subject. This determination will take into account various factors, including the response information, to arrive at an optimal dosing regimen for that subject.

As used herein, the term "optimal dosing regimen" means the dosing regimen determined by the medical practitioner to be optimal for a particular subject based on a variety of factors, including: the subject's age, size, and/or gender, the type of TBI, the potency of the toxin, and/or the subject's symptoms relating to TBI.

The invention also relates to a computer system programmed to perform steps of a computer-implemented method, the method comprising: receiving information related to a subject's response to treatment with botulinum neurotoxin; storing the response information into a database; and transmitting the response information to a medical practitioner. In an embodiment, the computer system comprises: a first computer device that is programmed to receive the response information; a second computer device on which the database resides; and a third computer device which receives the transmitted response information.

The invention further relates to a non-transitory computer-readable storage medium storing instructions that, when executed by a computer system, causes the computer system to perform steps of a computer-implemented method, the method comprising: receiving information related to a subject's response to treatment with botulinum neurotoxin;

storing the response information into a database; and transmitting the response information to a medical practitioner.

As used herein, the term "computer device" refers to any electronic device for storing and processing data, typically in binary form, according to instructions given to it in a software program, and includes, for example, a desktop, laptop, or tablet personal computers; "netbooks"; mobile communication devices, such as smartphones; personal digital assistants; portable audio or video file players; portable game players; portable electronic readers; or equivalent devices. The computer device can be in communication with another computer device by any suitable type of network (such as internet), and can use any suitable protocol, medium (e.g. fiber optic, coaxial cable, wireless broadband, etc.), network interface, or bandwidth.

The computer device used by the subject can be the same or different from the computer device used by the medical practitioner to access the response information. In some embodiments, the computer device used by the subject is the same computer device that the medical practitioner uses to access the response information. For example, the subject may enter the response information into his/her smartphone and bring that smartphone to the clinic visit to have the information viewed by the medical practitioner directly from the smartphone. In other embodiments, the computer device used to access the response information is different from the computer device used to enter the response information. For example, the response information may be entered into a website portal that is specially designed to collect this type of information and the medical practitioner uses his/her own desktop computer to access the website portal and view the information entered.

The computer device on which the database resides may be the same or different from the computer device used to receive the response information and/or the computer device used to access that information. In some embodiments, the computer device on which the database resides is different from both the computer device used to receive the response information and the computer device used by the medical practitioner to access that information. For example, the information may be entered into a website portal via a laptop computer. The information is stored on a web server computer, which is then accessed by the medical practitioner on his/her own computer to view the response information.

In one embodiment, the computer system is capable of determining a subsequent or optimal dosing regimen for the administration of botulinum neurotoxin to the subject. Such a determination may be made based on the response information with the appropriate adjustments in dosing amounts and frequency as discussed above.

In some embodiments, the invention is a software product. As used herein, the term "software product" refers to a non-transitory computer-readable storage medium storing instructions that when executed by a computer system, causes the computer system to perform the recited steps. The software product may reside on any suitable computer-readable storage medium, such as CD-ROM, DVD, memory, hard disk, flash drive, RAM, ROM, cache, and the like. The software platform for implementing the present invention can vary depending on design considerations such as user preference, cost, implementation, ease of use, machine capabilities, network limitations, etc.

In some embodiments, a computer system is provided. The computer system is programmed to perform steps of a computer-implemented method, the method comprising: receiving information associated with the subject's recovery; storing the information into a database; and transmitting the information to a medical practitioner.

In some embodiments, the invention is a computer system comprising one or more computer devices that are programmed to perform the methods of the present invention. The hardware platforms used by the subject, the medical practitioner, and/or any other third parties may be different, but operate together as a system. For example, the response information could be entered in one computer device, that information could be stored on a different computer device located remotely (e.g., a third party web server), and the medical practitioner could use his/her own computer device to access the information. In this scenario, these three computer devices can be considered to operate together as a system. The physical and/or functional components of the computer system may be distributed, centralized, or arranged in any suitable manner. Communications between the different physical and/or functional components may be performed in any suitable way. Moreover, the present invention encompasses all the various ways in which the operating work may be divided among different physical and/or functional components.

In some embodiments, the response information is received by the computer system from a first computer device, the database resides on a second computer device, and the response information is transmitted by the computer system to a third computer device. The computer devices may be separate. The first computer device may be in communication with the second computer device and the second computer device may be in communication with the third computer device. In one aspect, the first computer device is programmed to receive the response information from the subject, the database resides on a second computer device, and the third computer device receives the transmitted response information.

In other embodiments, a non-transitory computer-readable storage medium storing instructions is provided. The non-transitory computer-readable medium, when executed by a computer system, causes the computer system to perform steps of a computer-implemented method, the method comprising: receiving information associated with the subject's recovery; storing the response information into a database; and transmitting the response information to a medical practitioner.

The invention further relates in part to a composition for use in the treatment of a symptom of TBI in a subject. The composition comprises a therapeutically-effective amount of botulinum neurotoxin. In certain embodiments, the composition comprises a diluent, for example a sodium chloride solution. The solution may, for example, be a 0.9% sodium chloride solution. In other embodiments, the composition comprises botulinum neurotoxin as a lyophilized powder which may be reconstituted, for example using a sodium chloride solution.

In one embodiment, the composition comprises about 300 to 500 units of botulinum neurotoxin.

In one embodiment, the composition is formulated for the administration of botulinum neurotoxin parenterally, intra-muscularly, intradermally, or transdermally. In one embodiment, composition is formulated for parenteral administration, for example subcutaneous or intravenous administration. In one embodiment, botulinum neurotoxin is formulated for subcutaneous administration.

In one embodiment, the composition is formed by reconstituting lyophilized botulinum neurotoxin in a solution, for example the aforementioned sodium chloride solution. Following reconstitution, the composition may be stored under refrigeration at about 2 °C to about 8 °C and protected from light.

In one embodiment of the aforementioned method of treating a symptom of TBI, a composition as described above is administered to the subject. The amount of the composition administered may be the amount determined to be appropriate as described above.

In one embodiment, the composition further comprises an excipient. The excipient may, for example, be a filler, a binder, a disintegrant, an anti-adherent, a solvent, a buffering agent, a preservative, or a humectant. Examples of fillers include cellulose, lactose, sucrose, glucose, mannose, and sorbitol. The composition may comprise, for example, 10 mg/ml lactose. Examples of binders include gelatin, cellulose, polyvinyl pyrrolidone, starch, and sucrose. Examples of disintegrants include polyvinyl pyrrolidone and carboxymethyl cellulose. Examples of preservatives include parabens. Examples of solvents include water, oils, glycerol, propylene glycol, and ethanol. Examples of buffering agents include phosphates, carbonates, citrates, and lactates. Examples of humectants include glycerol, ethylene glycol, and polyethylene glycol (PEG).

It is to be understood that the present invention is not limited to the embodiments, aspects, or features described above, but encompasses any and all embodiments, aspects, and features within the scope of the following claims.

## SEQUENCE HOMOLOGY

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the art. Global methods align sequences from the beginning to the end of the molecule and determine the best alignment by adding up scores of individual residue pairs and by imposing gap penalties. Non-limiting methods include, e.g., CLUSTAL W, see, e.g., Julie D. Thompson et al., CLUSTAL W: Improving the Sensitivity of Progressive Multiple Sequence Alignment Through Sequence Weighting, Position- Specific Gap Penalties and Weight Matrix Choice, 22(22) Nucleic Acids Research 4673-4680 (1994); and iterative refinement, see, e.g., Osamu Gotoh, Significant Improvement in Accuracy of Multiple Protein. Sequence Alignments by Iterative Refinement as Assessed by Reference to Structural Alignments, 264(4) J. Mol. Biol. 823-838 (1996). Local methods align sequences by identifying one or more conserved motifs shared by all of the input sequences. Non-limiting methods include, e.g., Match-box, see, e.g., Eric Depiereux and Ernest Feytmans, Match-Box: A Fundamentally New Algorithm for the Simultaneous Alignment of Several Protein Sequences, 8(5) CABIOS 501 -509 (1992); Gibbs sampling, see, e.g., C. E. Lawrence et al., Detecting Subtle Sequence Signals: A Gibbs Sampling Strategy for Multiple Alignment, 262(5131 ) Science 208-214 (1993); Align-M, see, e.g., Ivo Van Walle et al., Align-M - A New Algorithm for Multiple Alignment of Highly Divergent Sequences, 20(9) Bioinformatics:1428-1435 (2004).

Thus, percent sequence identity is determined by conventional methods. See, for example, Altschul et al., Bull. Math. Bio. 48: 603-16, 1986 and Henikoff and Henikoff, Proc. Natl. Acad. Sci. USA 89:10915-19, 1992. Briefly, two amino acid sequences are aligned to optimize the alignment scores using a gap opening penalty of 10, a gap extension penalty of 1, and the "blosum 62" scoring matrix of Henikoff and Henikoff (ibid.) as shown below (amino acids are indicated by the standard one-letter codes).

The "percent sequence identity" between two or more nucleic acid or amino acid sequences is a function of the number of identical positions shared by the sequences. Thus, % identity may be calculated as the number of identical nucleotides / amino acids divided by the total number of nucleotides / amino acids, multiplied by 100. Calculations of % sequence identity may also take into account the number of gaps, and the length of each gap that needs to be introduced to optimize alignment of two or more sequences. Sequence comparisons and the determination of percent identity between two or more sequences can be carried out using specific mathematical algorithms, such as BLAST, which will be familiar to a skilled person.

ALIGNMENT SCORES FOR DETERMINING SEQUENCE IDENTITY

```
     A  R  N  D  C  Q  E  G  H  I  L  K  M  F  P  S  T  W  Y  V
A  4
R -1  5
N -2  0  6
D -2 -2  1  6
C  0 -3 -3 -3  9
Q -1  1  0  0 -3  5
E -1  0  0  2 -4  2  5
G  0 -2  0 -1 -3 -2 -2  6
H -2  0  1 -1 -3  0  0 -2  8
I -1 -3 -3 -3 -1 -3 -3 -4 -3  4
L -1 -2 -3 -4 -1 -2 -3 -4 -3  2  4
K -1  2  0 -1 -3  1  1 -2 -1 -3 -2  5
M -1 -1 -2 -3 -1  0 -2 -3 -2  1  2 -1  5
F -2 -3 -3 -3 -2 -3 -3 -3 -1  0  0 -3  0  6
P -1 -2 -2 -1 -3 -1 -1 -2 -2 -3 -3 -1 -2 -4  7
S  1 -1  1  0 -1  0  0  0 -1 -2 -2  0 -1 -2 -1  4
T  0 -1  0 -1 -1 -1 -1 -2 -2 -1 -1 -1 -1 -2 -1  1  5
W -3 -3 -4 -4 -2 -2 -3 -2 -2 -3 -2 -3 -1  1 -4 -3 -2 11
Y -2 -2 -2 -3 -2 -1 -2 -3  2 -1 -1 -2 -1  3 -3 -2 -2  2  7
V  0 -3 -3 -3 -1 -2 -2 -3 -3  3  1 -2  1 -1 -2 -2  0 -3 -1  4
```

The percent identity is then calculated as:

$$\frac{\text{Total number of identical matches}}{[\text{length of the longer sequence plus the number of gaps introduced into the longer sequence in order to align the two sequences}]} \times 100$$

Substantially homologous polypeptides are characterized as having one or more amino acid substitutions, deletions or additions. These changes are preferably of a minor nature, that is conservative amino acid substitutions (see below) and other substitutions that do not significantly affect the folding or activity of the polypeptide; small deletions, typically of one to about 30 amino acids; and small amino- or carboxyl-terminal extensions, such as an amino-terminal methionine residue, a small linker peptide of up to about 20-25 residues, or an affinity tag.

CONSERVATIVE AMINO ACID SUBSTITUTIONS

| | |
|---|---|
| Basic: | arginine |
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

In addition to the 20 standard amino acids, non-standard amino acids (such as 4-hydroxyproline, 6-N-methyl lysine, 2-aminoisobutyric acid, isovaline and $\alpha$ -methyl serine) may be substituted for amino acid residues of the polypeptides of the present invention. A limited number of non-conservative amino acids, amino acids that are not encoded by the genetic code, and unnatural amino acids may be substituted for polypeptide amino acid residues. The polypeptides of the present invention can also comprise non-naturally occurring amino acid residues.

Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins. For example, an in vitro system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell free system comprising an E. coli S30 extract and commercially available enzymes and other reagents. Proteins are purified by chromatography. See, for example, Robertson et al., J. Am. Chem. Soc. 113:2722, 1991; Ellman et al., Methods Enzymol. 202:301, 1991; Chung et al., Science 259:806-9, 1993; and Chung et al., Proc. Natl. Acad. Sci. USA 90:10145-9, 1993). In a second method, translation is carried out in Xenopus oocytes by microinjection of mutated mRNA and chemically aminoacylated suppressor tRNAs (Turcatti et al., J. Biol. Chem. 271:19991-8, 1996). Within a third method, E. coli cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the polypeptide in place of its natural counterpart. See, Koide et al., Biochem. 33:7470-6, 1994. Naturally occurring amino acid residues can be converted to non-naturally occurring species by in vitro chemical modification. Chemical modification can be combined with site-directed mutagenesis to further expand the range of substitutions (Wynn and Richards, Protein Sci. 2:395-403, 1993).

A limited number of non-conservative amino acids, amino acids that are not encoded by the genetic code, non-naturally occurring amino acids, and unnatural amino acids may be substituted for amino acid residues of polypeptides of the present invention.

Essential amino acids in the polypeptides of the present invention can be identified according to procedures known in the art, such as site-directed mutagenesis or alanine-scanning mutagenesis (Cunningham and Wells, Science 244: 1081-5, 1989). Sites of biological interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-12, 1992;

Smith et al., J. Mol. Biol. 224:899-904, 1992; Wlodaver et al., FEBS Lett. 309:59-64, 1992. The identities of essential amino acids can also be inferred from analysis of homologies with related components (e.g. the translocation or protease components) of the polypeptides of the present invention.

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide the skilled person with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure.

Amino acids are referred to herein using the name of the amino acid, the three letter abbreviation or the single letter abbreviation. The term "protein", as used herein, includes proteins, polypeptides, and peptides. As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme". The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

Other definitions of terms may appear throughout the specification. Before the exemplary embodiments are described in more detail, it is to be understood that this disclosure is not limited to particular embodiments described, and as such may vary. It is also to be understood that the terminology used herein is for the purpose of describing particular embodiments only, and is not intended to be limiting, since the scope of the present disclosure will be defined only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "a toxin" includes a plurality of such candidate agents and reference to "the toxin" includes reference to one or more toxins and equivalents thereof known to those skilled in the art, and so forth.

In describing the invention, where a range of values is provided with respect to an embodiment, it is understood that each intervening value is encompassed within the embodiment.

The publications discussed herein are provided solely for their disclosure prior to the filing date of the present application. Nothing herein is to be construed as an admission that such publications constitute prior art to the claims appended hereto.

## BRIEF DESCRIPTION OF THE DRAWINGS

Embodiments of the invention will now be described, by way of example only, with reference to the following Figures and Examples.

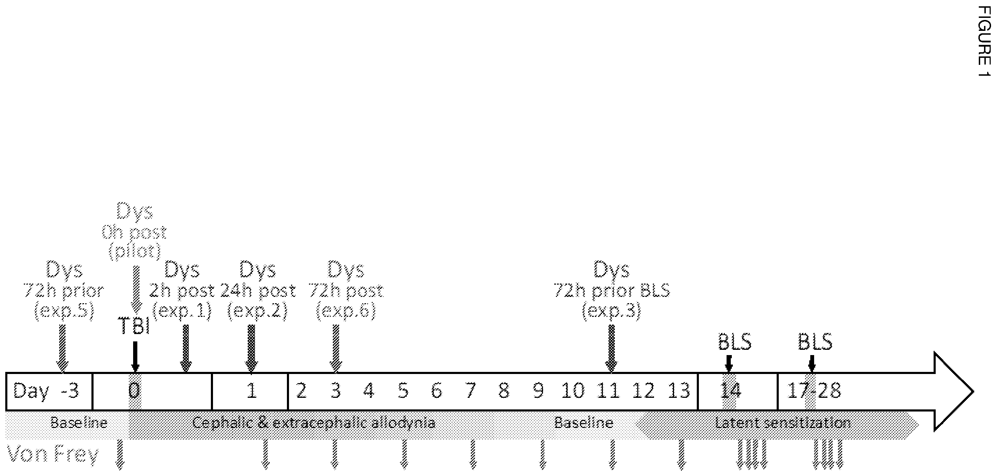

**Figure 1** shows a schematic of the experimental time-line for the pilot study (green), the originally proposed main study (exp.1-3, red) and additional experiments (exp. 5-6, blue).

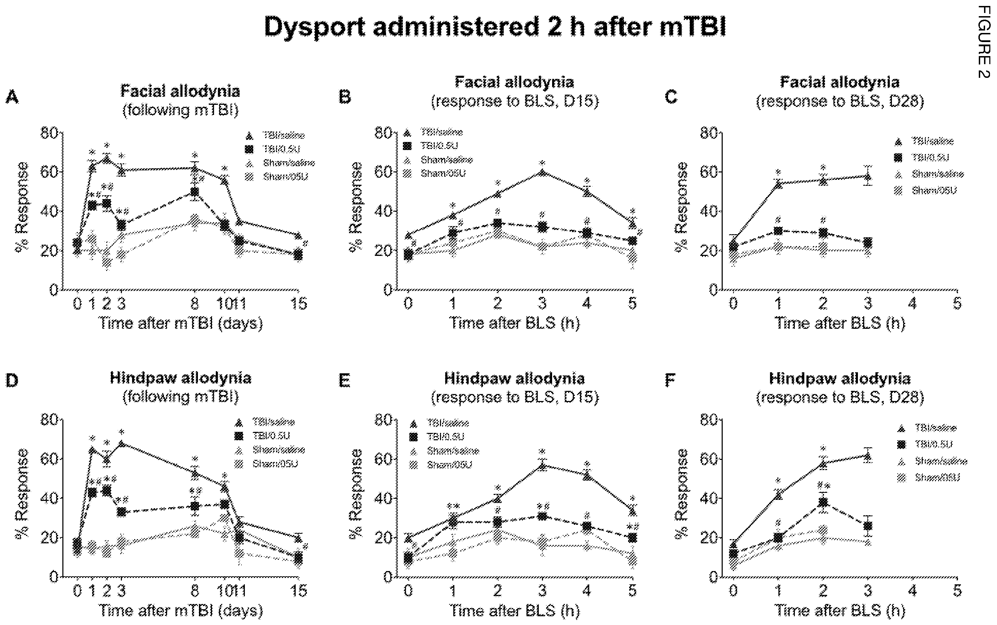

**Figure 2** shows Dysport administered 2 hours after mTBI prevents acute mTBI-induced allodynia and latent BLS-induced allodynia. After measuring baseline periorbital and hindpaw responses mice were given mTBI or sham injury and 2 h following the injury/sham-injury were administered saline or Dysport (0.5 U). (A) Periorbital and (D) hindpaw responses were measured in mice periodically over 15 days after mTBI or sham injury. At day 15, all mice were exposed to BLS and (B) periorbital and (E) hindpaw tactile allodynia was measured over a 5-h time course. At day 28, all mice were again exposed to BLS and (C) periorbital and (F) hindpaw tactile allodynia was measured over a 3-h time course. Two-way repeated measures ANOVA with Sidak's multiple comparison test shows significantly elevated frequency of tactile responses in the mTBI/saline group compared to sham/saline mice at times indicated by red asterisk (*), and significantly higher responses in the mTBI/0.5U group compared to sham/0.5U mice indicated by blue asterisk (*). Allodynia was significantly reduced in the mTBI/0.5U group compared to mTBI/saline group at times indicated by blue hashtag (#). Data are plotted as means and SEM; N = 5-10 mice/group.

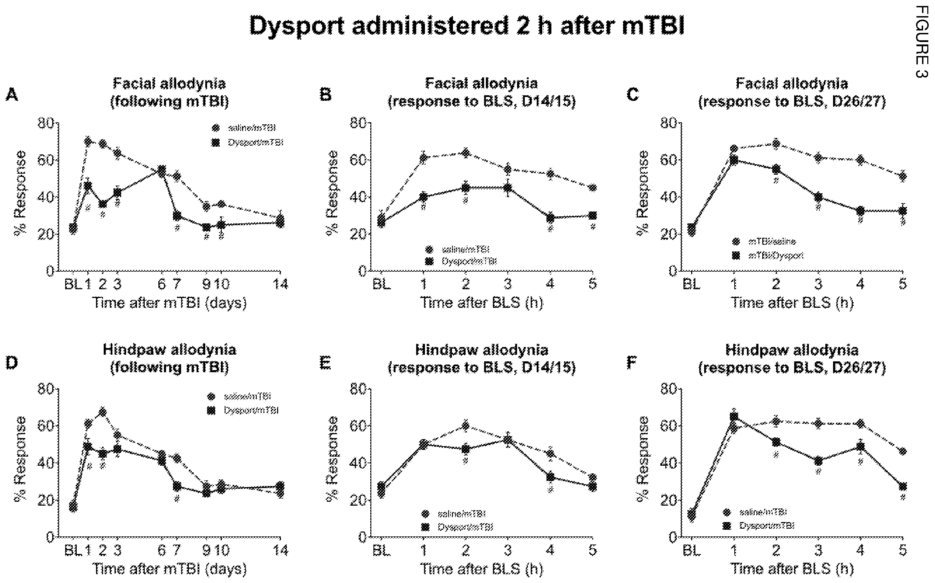

**Figure 3** shows Dysport administered 2 hours after mTBI prevents acute mTBI-induced allodynia and latent BLS-induced allodynia. After measuring baseline periorbital and hindpaw responses mice were given mTBI injury and 2 h following the injury mice were administered saline or Dysport (0.5 U). (A) Periorbital and (D) hindpaw responses were measured in mice periodically over 14 days. At day 14/15, all mice were exposed to BLS and (B) periorbital and (E) hindpaw tactile allodynia was measured over a 5-h time course. At day 26/27, all mice were again exposed to BLS and (C) periorbital and (F) hindpaw tactile allodynia was measured over a 5-h time course. Two-way repeated measures ANOVA with Sidak's multiple comparison test shows significantly reduced frequency of tactile responses in the mTBI/0.5U group compared to mTBI/saline group at times indicated by blue hashtag (#). Data are plotted as means and SEM; N = 8 mice/group.

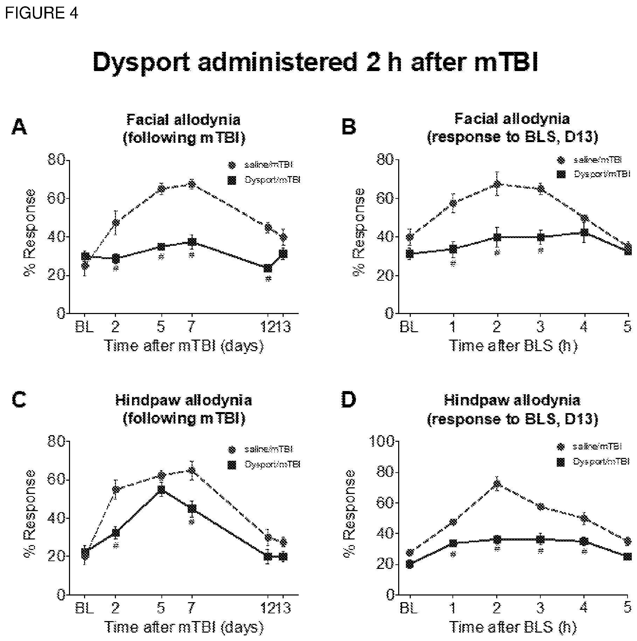

**Figure 4** shows Dysport administered 2 hours after mTBI prevents acute mTBI-induced allodynia and latent BLS-induced allodynia. After measuring baseline periorbital and hindpaw responses mice were given mTBI injury and 2 h following the injury mice were administered saline or Dysport (0.5 U). (A) Periorbital and (D) hindpaw responses were measured in mice periodically over 14 days. At day 14/15, all mice were exposed to BLS and (B) periorbital and (E) hindpaw tactile allodynia was measured over a 5-h time course. At day 26/27, all mice were again exposed to BLS and (C) periorbital and (F) hindpaw tactile allodynia was measured over a 5-h time course. Two-way repeated measures ANOVA with Sidak's multiple comparison test shows significantly reduced frequency of tactile responses in the mTBI/0.5U group compared to mTBI/saline group at times indicated by blue hashtag (#). Data are plotted as means and SEM; N = 8 mice/group.

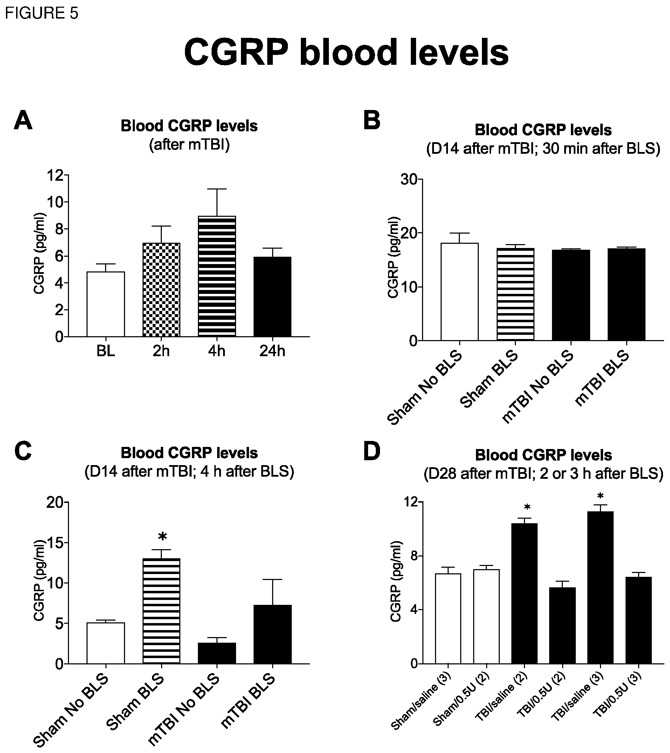

**Figure 5** shows levels of CGRP in blood. (A) Blood was collected from naïve (BL) mice and at indicated times after mTBI injury and CGRP levels in plasma were analyzed. One way ANOVA shows no significant difference between groups. N = 4 mice/group. (B) Mice were given mTBI or sham injury, development of allodynia in mTBI but not sham mice was verified. On day 14 mice were exposed to BLS or no stress as indicated and blood was collected 30 minutes after BLS. CGRP levels in plasma were analyzed. One way ANOVA shows no significant difference between groups. N = 3-4 mice/group. (C) Mice were given mTBI or sham injury, development of allodynia in mTBI but not sham mice was verified. On day 14 mice were exposed to BLS or no stress as indicated and blood was collected 4 hours after BLS. CGRP levels in plasma were analyzed. One way ANOVA with Tukey's post test shows significant difference between Sham/No BLS and Sham/BLS groups. N = 3 mice/group. (D) Mice were given mTBI or sham injury, and were administered saline or Dysport 2 h post injury. Development of allodynia in the mTBI/saline group and decreased allodynia in the other groups was verified (See Figure 2). On day 15, all mice were exposed to BLS. On day 28 all mice were again exposed to BLS and blood was collected 2-3 hours after BLS as indicated. CGRP levels in plasma were analyzed. One way ANOVA with Tukey's post test shows significant difference between Sham/saline and mTBI/saline groups. N = 8 mice/group.

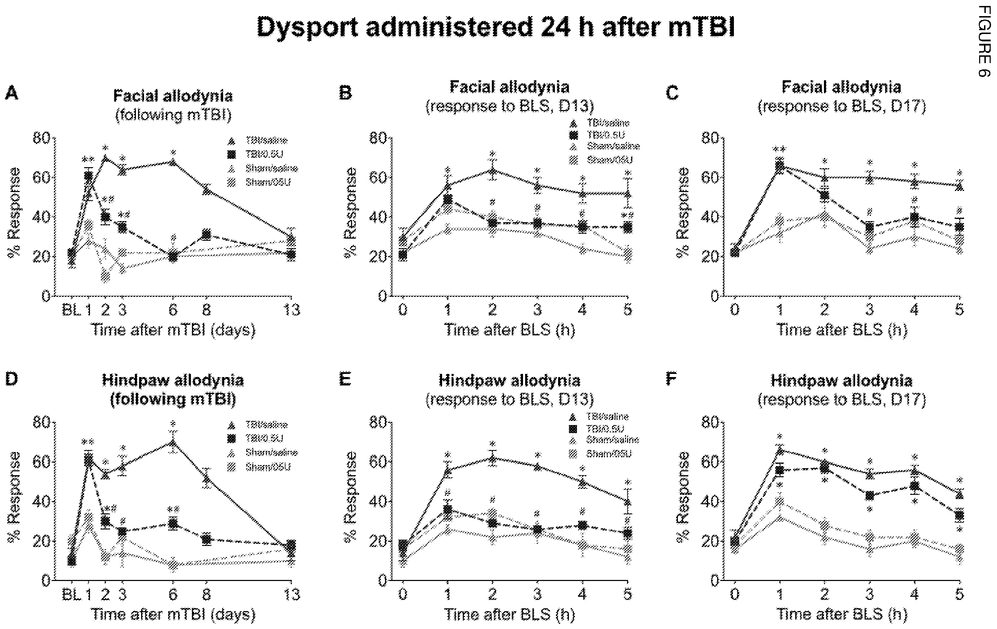

**Figure 6** shows Dysport administered 24 hours after mTBI reverses acute mTBI-induced allodynia and prevents latent BLS-induced allodynia. After measuring baseline periorbital and hindpaw responses, mice were given mTBI or sham injury and (A) periorbital and (D) hindpaw tactile responses were measured 24 h later. Following the Day 1 measurements, mice were administered saline or Dysport (0.5 U) and tactile responses were measured periodically over 13 days after mTBI or sham injury. At day 13, all mice were exposed to BLS and (B) periorbital and (E) hindpaw tactile allodynia was measured over a 5-h time course. At day 17, all mice were again exposed to BLS and (C) periorbital and (F) hindpaw tactile allodynia was measured over a 5-h time course. Two-way repeated measures ANOVA with Sidak's multiple comparison test shows significantly elevated frequency of tactile responses in the mTBI/saline group compared to sham/saline mice at times indicated by red asterisk (*), and significantly higher responses in the mTBI/0.5U group compared to sham/0.5U mice indicated by blue asterisk (*). Allodynia was significantly reduced in the mTBI/0.5U group compared to mTBI/saline group at times indicated by blue hashtag (#). Data are plotted as means and SEM; N = 5-10 mice/group.

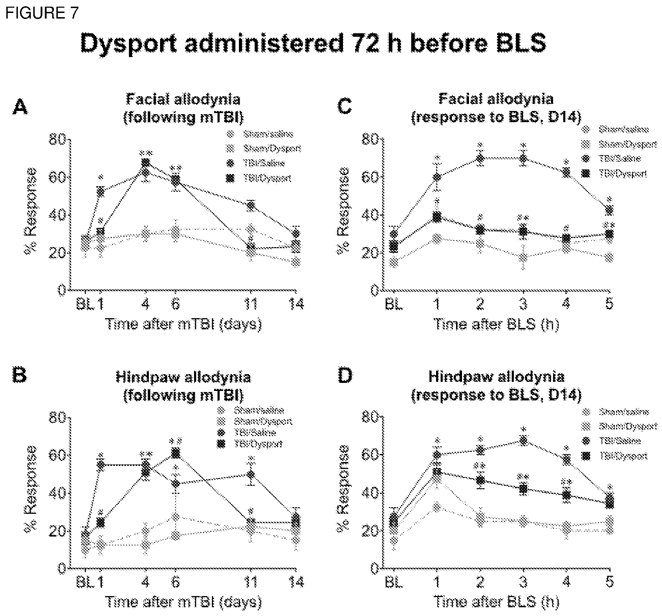

**Figure 7** shows Dysport administered on day 11 after mTBI (72 hours before BLS) prevents latent BLS-induced periorbital allodynia. Baseline periorbital (A) and hindpaw (B) responses were measured; mice were then given mTBI or sham injury and tactile responses were measured at Days 1, 4, 6 and 11 after the injury. Following the Day 11 measurements, mice were administered saline or Dysport (0.5 U) and tactile responses were measured on Day 14. At day 14, all mice were exposed to BLS and periorbital (C) and hindpaw (D) tactile allodynia was measured over a 5-h time course. Two-way repeated measures ANOVA with Sidak's multiple comparison test shows significantly elevated frequency of tactile responses in the mTBI/saline group compared to sham/saline mice at times indicated by red asterisk (*), and significantly higher responses in the mTBI/0.5U group compared to sham/0.5U mice indicated by blue asterisk (*). Allodynia was significantly different between the mTBI/0.5U and mTBI/saline groups at times indicated by blue hashtag (#). Data are plotted as means and SEM; N = 4-9 mice/group.

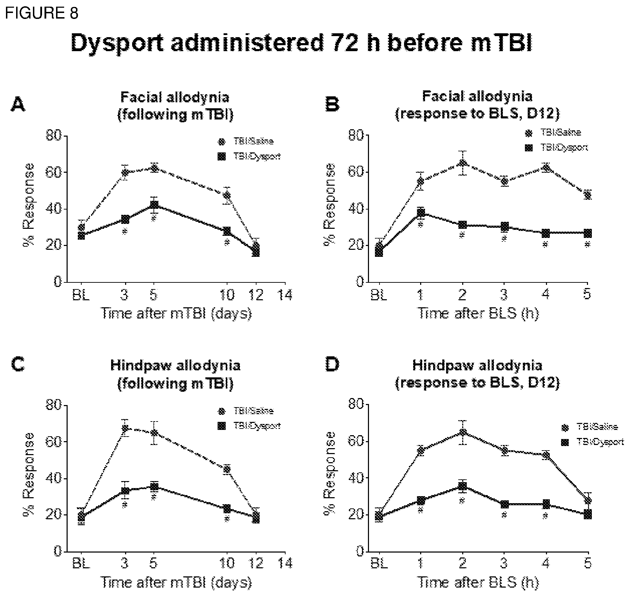

**Figure 8** shows Dysport administered 72 hours before mTBI prevents acute mTBI-induced allodynia and latent BLS-induced allodynia. Mice were pre-treated with saline or Dysport (0.5 U); 3 days (72 h) later (A) periorbital and (C) hindpaw responses were measured and mice were given mTBI injury and tactile responses were assessed periodically over 12 days. At day 12, all mice were exposed to BLS and (B) periorbital and (D) hindpaw tactile allodynia was measured over a 5-h time course. Two-way repeated measures ANOVA with Sidak's multiple comparison test shows significantly reduced frequency of tactile responses in the mTBI/Dysport group compared to mTBI/saline group at times indicated by blue hashtag (#). Data are plotted as means and SEM; N = 4 saline and 9 Dysport mice.

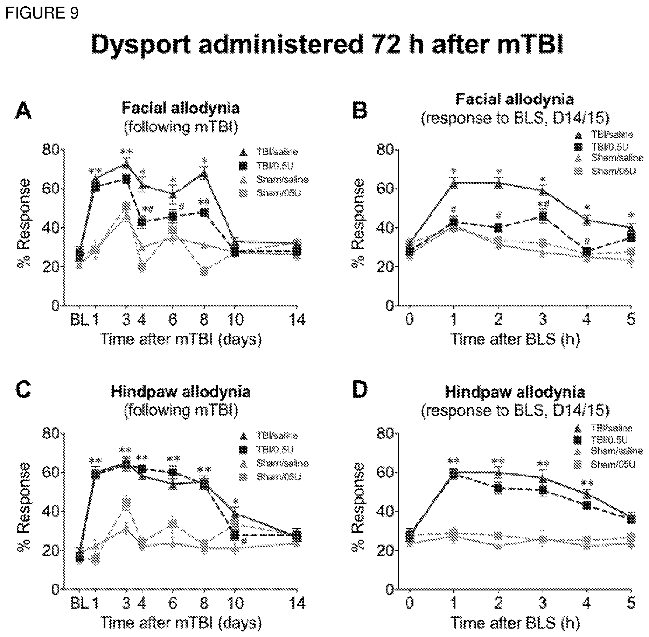

**Figure 9** shows Dysport administered 72 hours after mTBI partially reverses acute mTBI-induced periorbital allodynia and prevents latent BLS-induced periorbital allodynia. Baseline periorbital (A) and hindpaw (C) responses were measured; mice were then given mTBI or sham injury and tactile responses were measured at Day 1 and Day 3 after the injury. Following the Day 3 measurements, mice were administered saline or Dysport (0.5 U) and tactile responses were measured periodically over 14 days after mTBI or sham injury. At day 14, all mice were exposed to BLS and (B) periorbital and (D) hindpaw tactile allodynia was measured over a 5-h time course. Two-way repeated measures ANOVA with Sidak's multiple comparison test shows significantly elevated frequency of tactile responses in the mTBI/saline group compared to sham/saline mice at times indicated by red asterisk (*), and significantly higher responses in the mTBI/0.5U group compared to sham/0.5U mice indicated by blue asterisk (*). Allodynia was significantly reduced in the mTBI/0.5U group compared to mTBI/saline group at times indicated by blue hashtag (#). Data are plotted as means and SEM; N = 8-10 mice/group.

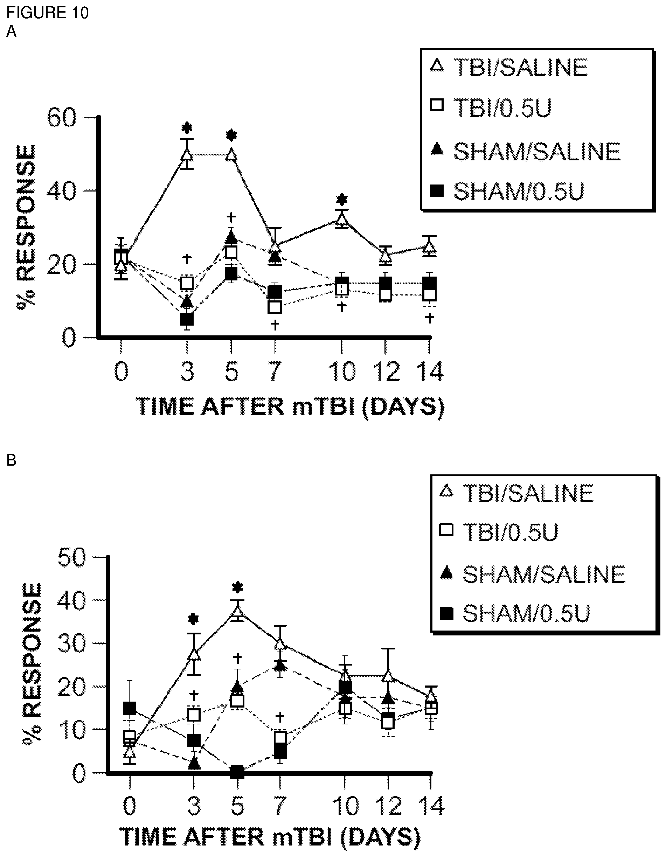

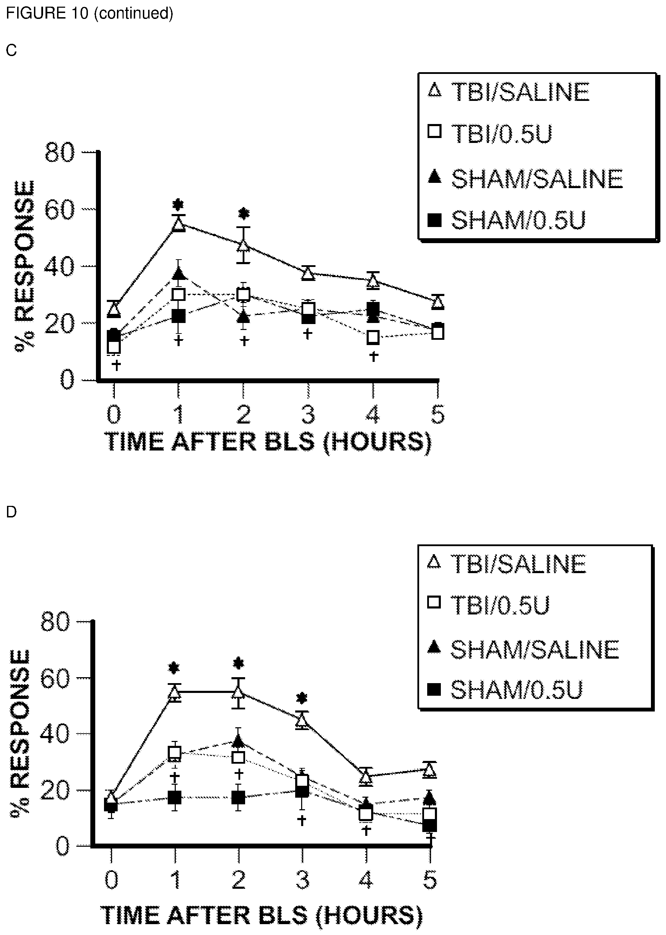

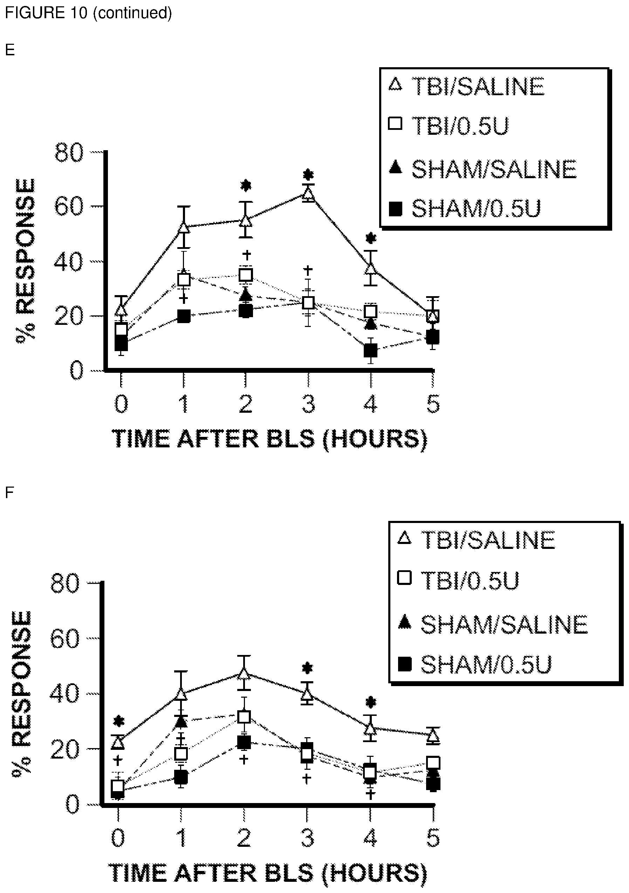

**Figure 10A-F** depicts the effect of DYSPORT® (0.5 units/mouse) treatment in mice when DYSPORT® is administered immediately after mild traumatic brain injury (mTBI). Facial allodynia (Fig. 10A) and hindpaw allodynia (Fig. 10B) are measured 0, 3, 5, 7, 10, 12, and 14 days post-injury. Facial alloydynia (Fig. 10C) and hindpaw allodynia (Fig. 10D) were measured again for 5 hours following bright light stress (BLS) on day 15 post-injury. Facial alloydynia (Fig. 10E) and hindpaw allodynia (Fig. 10F) were also measured again for 5 hours following further exposure to BLS on day 32 post-injury. Asterisks denote statistically significant differences between the TBI/saline and sham/saline groups and crosses denote statistically significant differences between the TBI/saline and TBI/0.5 U DYSPORT® groups (p<0.05).

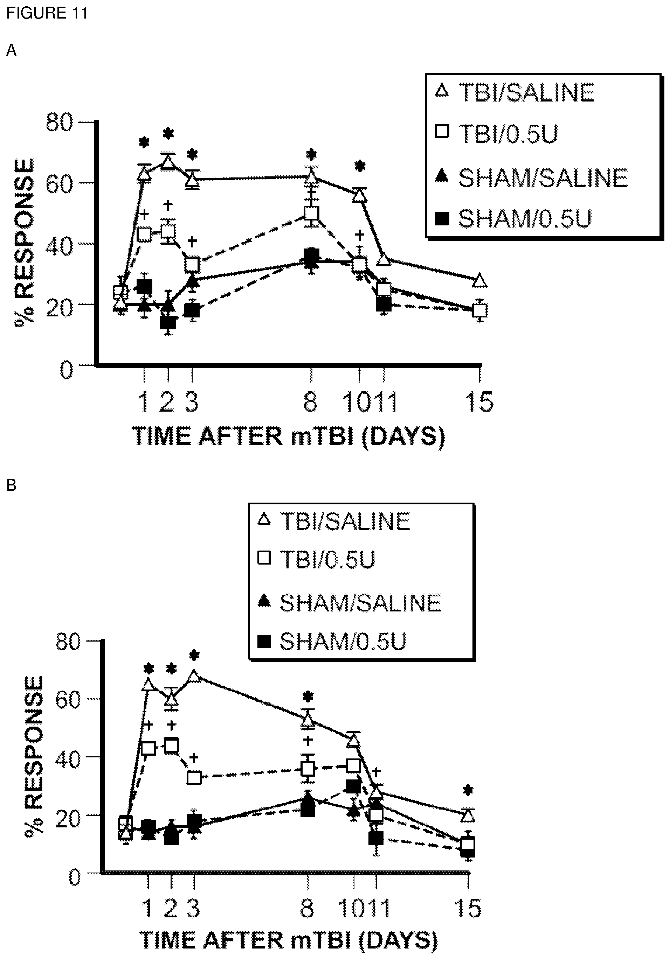

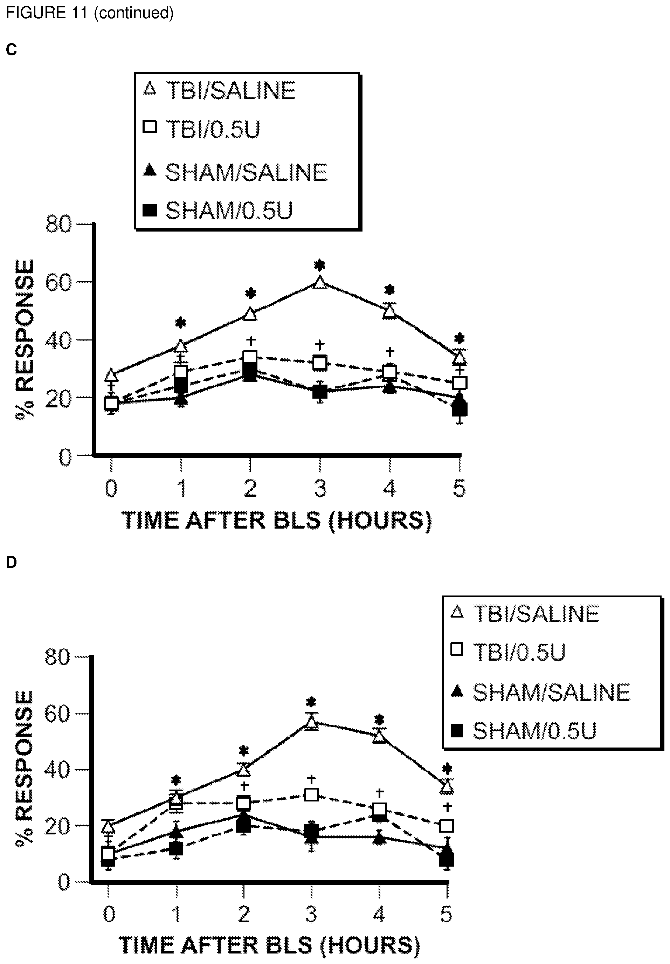

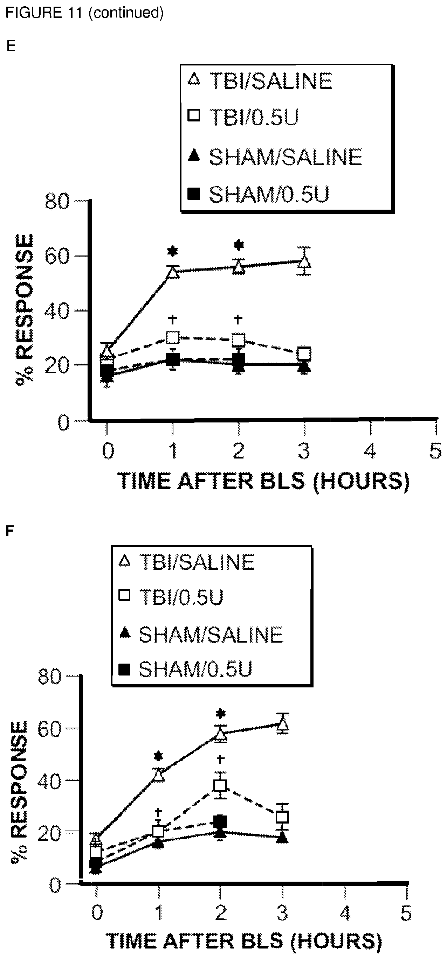

**Figure 11 A-F** depicts the effect of DYSPORT® (0.5 units/mouse) treatment when DYSPORT® is administered two hours after mild traumatic brain injury (mTBI). Facial allodynia (Fig. 11A) and hindpaw allodynia (Fig. 11B) are measured 0, 1, 2, 3, 8, 10, 11, 15 days post-injury. Facial alloydynia (Fig. 11C) and hindpaw allodynia (Fig. 11D) were measured again for 5 hours following bright light stress (BLS) on day 15 post-injury. Facial alloydynia (Fig. 11E) and hindpaw allodynia (Fig. 11F) were also measured again for 3 hours following further exposure to BLS on day 28 post-injury. Asterisks denote statistically significant differences between the TBI/saline and sham/saline groups and crosses denote statistically significant differences between the TBI/saline and TBI/0.5 U DYSPORT® groups (p<0.05).

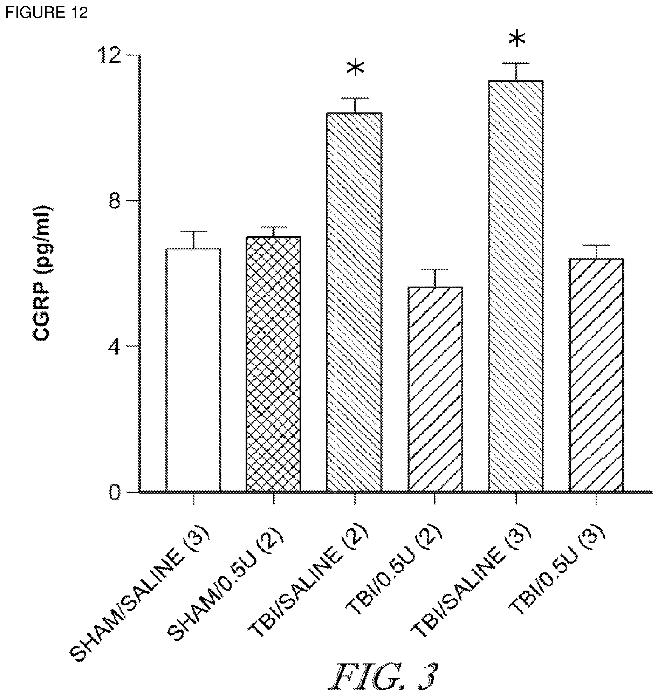

**Figure 12** depicts the effects of DYSPORT® (0.5 U/mouse) administration two hours after mild traumatic brain injury (mTBI) on blood concentrations of CGRP 2 or 3 hours after exposure to bright light stress (BLS) on post-TBI day 28. *p<0.05 in comparison to the corresponding TBI/saline group.

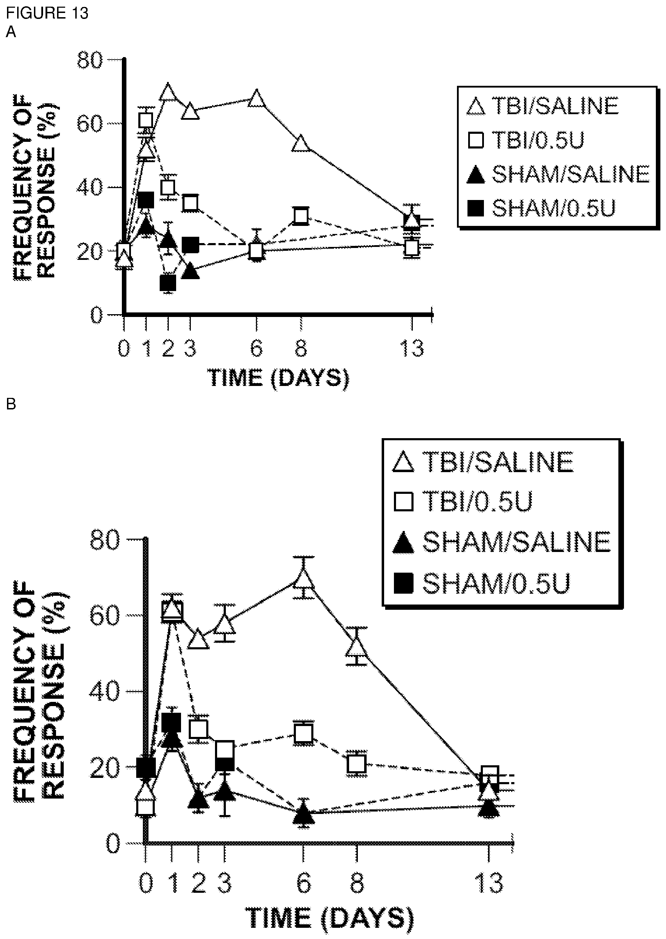

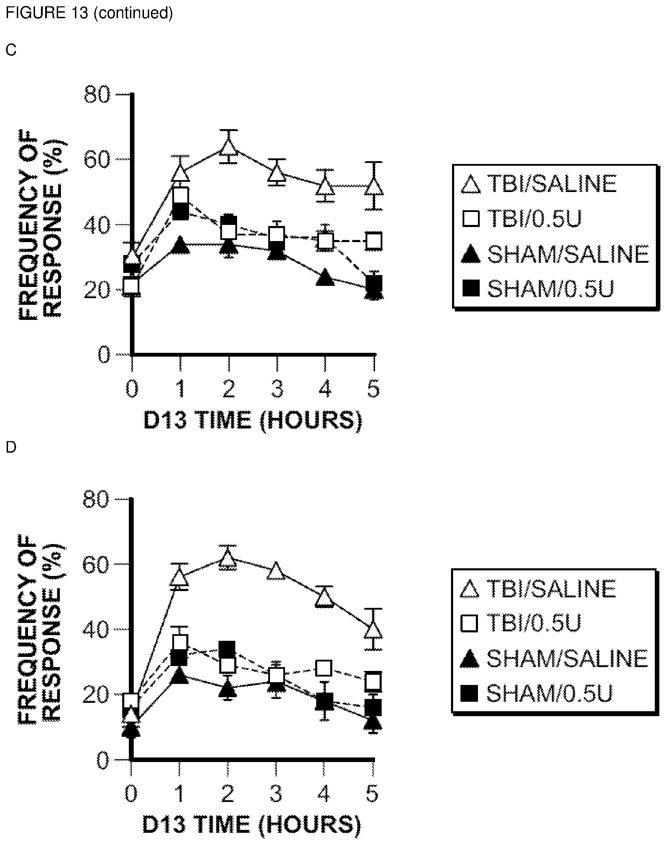

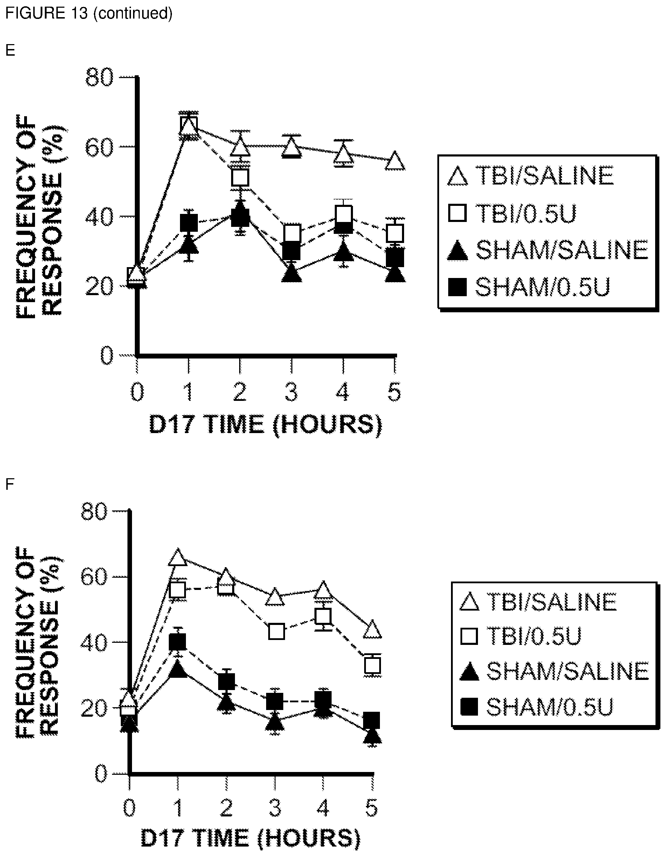

**Figure 13** depicts the effect of DYSPORT® (0.5 units/mouse) treatment when DYSPORT® is administered 24 hours after mild traumatic brain injury (mTBI). Facial allodynia (Fig. 13A) and hindpaw allodynia (Fig. 13B) are measured 0, 1, 2, 3, 6, 8, and 13 days post-injury. Facial alloydynia (Fig. 13C) and hindpaw allodynia (Fig. 13D) were measured again for 5 hours following bright light stress (BLS) on day 15 post-injury. Facial alloydynia (Fig. 12E) and hindpaw allodynia (Fig. 12F) were also measured again for 5 hours following further exposure to BLS on day 17 post-injury.

## EXAMPLES

The invention will now be described, by way of example only, with reference to the following Examples.

Summary:

Presented here are the results of a study in a preclinical model of TBI (e.g. post-traumatic headache). Three main experiments were conducted to determine if acute administration of BoNT/A (DYSPORT®) after a single mTBI blocks the expression of mTBI-induced cephalic pain (Aim 1) and to characterize the possible efficacy of DYSPORT® treatment after the initial cephalic pain has resolved, but latent sensitization to environmental stressors demonstrated as post traumatic headache (PTH) is still present (Aim 2).

Further experiments tested if pre-treatment of mice with DYSPORT® three days (72h) prior to mTBI (Exp. 5) can prevent pain symptoms and 2) if treatment with DYSPORT® 72h after mTBI can reverse established mTBI-induced allodynia in mTBI animals (Exp 6). These experiments have generated important insights into the neural mechanisms of mTBI-associated allodynia and the mechanisms of anti-allodynic actions of DYSPORT®. Experiment 5 established an efficacy of DYSPORT® as a preventive treatment during activities where mTBI injuries are likely, including competitive sports or military mTBI injuries (e.g., falls). Experiment 6 widened a therapeutic window for DYSPORT® efficacy after the mTBI.

Introduction:

Traumatic brain injury (TBI) is a serious public health issue that can result from sports or accidents in the general population or from combat activities in military personnel. Sports-related (e.g., football, soccer, ice hockey) TBI is very common and negatively impacts the lives of youth, collegiate and professional male and female athletes. Such TBI is commonly described as mild (mTBI). Mild TBI exhibits little or no apparent damage but is thought to cause direct injury to the skull, brain, meninges, vasculature, or neck with different severity and manifestations, which can result in psychiatric, cognitive, and physiological disorders later in life. A complicating factor is that successive injuries, including sub-concussive ones, may synergize with previous ones resulting in collectively greater effects on neurological and cognitive consequences.

Following mTBI, patients can experience a sustained and unrelenting headache that often has migrainous features including pain, and hypersensitivity to light, sound and touch (i.e., photophobia, phonophobia and allodynia). This post-traumatic headache (PTH) is debilitating and can be continuous or episodic with initiation of the headache linked to life events including exercise and most significantly, stress. These events are common as athletes return to competition, the classroom or work environments.

At present, therapies for TBI and for PTH have not been clinically validated. A critical impediment to the discovery of effective and well-tolerated therapeutics for PTH has been a relative lack of understanding of the molecular, biochemical and physiological mechanisms of this disorder.

The inventors utilised a mouse model of single or repetitive mild TBI (rmTBI) injury where mice experience mild, closed head injury without resistance and without observable extracranial damage. This model produces symptoms that are consistent with migraine-like pain that is commonly experienced following single or repeated mild concussive injuries. This model has several important advantages: (a) the forces can be controlled without producing observable damage to the skull and to the underlying brain tissues; (b) the model allows for the assessment of the consequences of repeated head impacts modeling the repetitive nature of concussive injuries experienced by many athletes; and (c) this model closely mimics the most critical factors in producing mild concussive brain injuries, which are high velocity impact and rapid head acceleration and rotation. These studies have shown that this approach can mimic PTH for up to 60 days following a single mTBI. In this model, mice previously exposed to a single mTBI demonstrate hypersensitivity to stress and to light, consistent with clinical experience. The availability of this model allows assessment of potential efficacy of pharmacological interventions in PTH.

Objectives:

- Determine if acute administration of DYSPORT® after a single mTBI blocks the expression of mTBI-induced cephalic pain and prevents development of latent sensitization revealed by stress-induced reinstatement of cephalic allodynia.

- Characterize the efficacy of DYSPORT® treatment after the initial cephalic allodynia has resolved, but latent sensitization to environmental stressors demonstrated as post traumatic headache (PTH) is still present.

Materials and Methods:

Animals

Male, C57Bl/6J adult mice weighing 17-22 grams were housed five to a cage on a 14/10-hour light/dark cycle (5 am-7 pm lights on) with food and water ad libitum. Experiments were conducted during the light cycle phase. All experiments were performed in accordance with the ARRIVE reporting guidelines and with the approval of the Mayo Clinic Institutional Animal Care and Use Committee and Teva Pharmaceuticals. Group size requirements to obtain significance at the $\alpha$=0.05 and statistical power 0.9 were determined from previous experiments using power analysis.

Induction of Mild Traumatic Brain Injury

The mouse model of experimental mTBI was adapted from Kane et al (J Neurosci Methods 2012; 203: 41-49. DOI: 10.1016/j.jneumeth.2011.09.003). Briefly, mice were lightly anaesthetized with 3% isofluorane and then laid with their ventral surface on an elevated tissue paper stage capable of supporting body weight with the head unrestrained. The paper stage was situated over a plexiglass apparatus with a soft sponge at the bottom. A metal guide tube was directed to the top of the mouse skull between the ears to ensure standardized placement of the weighted drop. The weight (100 g), released from a height of 94 cm, results in a concussive impact to the head, pushing the mouse through the tissue paper and flipping it down to land on the soft sponge. All mTBI mice in this study experienced both rotational and linear head forces. After impact the weight falls through the apparatus thereby avoiding a second impact with the animal. Following the procedure, mice were returned to their home cages and allowed to recover. Sham animals were anaesthetized and placed on the tissue paper stage but did not undergo the weighted drop or rotational flip. All mice awoke within 5 minutes of the procedure and were observed to confirm that no visual signs of neurological complications arose. Animals remained grouped in their same cohorts following the procedure.

Bright Light Stress (BLS) Challenge

Unrestrained mice were exposed to BLS induced by LED strips (1000 lux output) that were placed on both sides of their home Plexiglass cages for 15 minutes. The parameters of BLS protocol were modified from our previous studies in Sprague Dawley rats to produce mild stress that arises from endogenous mechanisms and does not elicit significant CA in naïve or sham mice.

DYSPORT® Administration

DYSPORT® was provided by Ipsen and was diluted with saline to concentration of 0.5U/50 μl. One injection of 0.5U/50 μl DYSPORT® or vehicle (saline) was administered subcutaneously along the lambdoid suture at indicated times following the mTBI or sham injury. This dose and route of administration was well tolerated and the animals did not show any visible adverse effects.

Behavioral Assessment of Cutaneous Allodynia

Prior to baseline behavioral assessment, mice were placed individually in elevated Plexiglass chambers with mesh flooring, located in a quiet, non-trafficked area and allowed to acclimate for 3 days for 2 hours. Starting on day 0 (pre-mTBI baseline) and periodically thereafter, cephalic (periorbital) and extracephalic (hindpaw) allodynia was measured in the same mice following a 2-hour acclimation period. For assessment of periorbital allodynia, a 0.4 g (3.61) von Frey filament was applied with just enough pressure to cause the filament to display a slight arch to the periorbital region 10 times with a space of 20-30 sec between each application. A positive response was considered swiping of the face, shaking of the head, and/or turning away from the stimuli. Running away or rearing up were not considered as positive responses. For assessment of hindpaw allodynia, a 0.6 g (3.84) von Frey filament was applied with just enough pressure to cause the filament to display a slight arch to the left hindpaw 10 times with a space of 20-30 sec between each application. Sharp withdrawal of the paw, shaking and/or licking the paw were considered a positive response, while lifting of the paw with the filament or running away were not. Frequency response was calculated as [(number of positive responses/10) * 100%]. Following mTBI induction, CA was measured for a time course of up to 14 days and in response to DYSPORT® and control/vehicle administration.

CGRP measurements

Concentrations of CGRP in plasma were measured with commercially purchased mouse enzyme-linked immunosorbent assay kits (Cayman Chemical, Ann Harbor, MI, USA). Mice were anesthetized and whole blood was collected from the heart at indicated times and stored in ethylenediaminetetraacetic acid-coated vials. Blood was centrifuged at 3000 rpm for 20 minutes at 4°C in order to separate the plasma. Plasma was stored at -80°C and analyzed within 1 week of collection.

Study design

Aim 1: Determine if acute administration of DYSPORT® after a single mTBI prevents the expression of mTBI-induced cephalic pain and latent sensitization revealed by stress-induced allodynia. Efficacy of DYSPORT® was assessed at four different acute time-points following mTBI: 1) immediately after mTBI (Exp. 5); 2) 2 h after mTBI (Exp. 1); 3) 24 h after mTBI (Exp. 2); 4) 72 h after mTBI (Exp. 6).

Aim 2: Characterize the possible efficacy of DYSPORT® treatment after the initial cephalic pain has resolved, but latent sensitization to environmental stressors demonstrated as post traumatic headache (PTH) is present (Exp. 3).

## EXAMPLE 1 - Experiments 1-7

### Experiment 1 (outline of experiment):

DYSPORT® (0.5 U/mouse) was administered at 2 hours following the mTBI.

A. Baseline cutaneous allodynia was determined for male, C57/Bl6 mice using calibrated von Frey filaments. Following mTBI and DYSPORT® administration, cutaneous allodynia was followed intermittently for 14 days.
B. On day 15, mice were exposed to environmental stress (bright light stress, BLS) for 15 min and cutaneous allodynia was assessed over a 5h time period following BLS.
C. On day 28 the mice were again exposed to BLS for 15 min followed by assessment of cutaneous allodynia over a 4h time period. On day 29, the animals were exposed to the same BLS protocol, blood was collected at the peak of post BLS cutaneous allodynia. Plasma was prepared and used to estimate levels of CGRP using CGRP EIA kit (Phoenix Pharmaceuticals, Inc.).
D. In a separate cohort of animals exposed to mTBI and DYSPORT®, blood was collected 4h and 26 h post mTBI (i.e.; 2 h and 24 h post DYSPORT®) and the levels of CGRP in plasma were analyzed.

### Experiment 2 (outline of experiment):

DYSPORT® (0.5 U/mouse) was administered at 24h following the mTBI. Experiments 2A-D was performed in the same manner as the Experiment 1 (1A-D), but DYSPORT® was administered 24h after induction of mTBI when neuronal changes (increased CGRP levels) and cutaneous allodynia are likely to be developed.

### Experiment 3 (outline of experiment):

DYSPORT® (0.5 U/mouse) was administered at day 11 following the mTBI, when von Frey responses have returned to baseline and we investigated if on day 14, BLS-induced allodynia would be blocked.

A. On day 14 following the TBI, cutaneous allodynia will be determined before BLS and during a 5h time period following BLS. BoNT-A will be administered at the end of the 4h period.
B. On day 21, cutaneous allodynia will be measured before BLS and during a 5h time period following BLS.
C. The following day (day 22), the animals will be exposed to the same BLS protocol, blood will be collected at the peak of post BLS cutaneous allodynia (determined on day 21) and the levels of CGRP will be determined.

### Experiment 4 (outline of experiment):

DYSPORT® will be administered at 2h following the TBI using subcutaneous administration route (results omitted). The dose, volume, number of injections and the site will be provided by Ipsen.

A. Baseline cutaneous allodynia will be determined for all mice using calibrated von Frey filaments. Following TBI and BoNT-A administration, cutaneous allodynia will be followed daily (work days) for 14 days.

B. At day 15 we will expose the mice to environmental stress (bright light stress, BLS) for 15 min and assess cutaneous allodynia over a 4h time period following BLS.

### Experiment 5 (outline of experiment):

DYSPORT® (0.5 U/mouse) was administered 3 days (72h) before the mTBI.

A. Baseline cutaneous allodynia was determined and mice were exposed to mTBI or sham injury. Following mTBI, cutaneous allodynia was followed intermittently for 12 days.
B. At day 12, mice were exposed to environmental stress (bright light stress, BLS) for 15 min and cutaneous allodynia was assessed over a 5h time period following BLS.

### Experiment 6 (outline of experiment):

DYSPORT® (0.5 U/mouse) was administered immediately (at 0 hours) following the mTBI (before the animals wake up from anesthesia) (results omitted).

A. Baseline cutaneous allodynia was determined for male, C57/Bl6 mice using calibrated von Frey filaments. Following mTBI and DYSPORT® administration, cutaneous allodynia was followed intermittently for 14 days.
B. At day 15, mice were exposed to environmental stress (bright light stress, BLS) for 15 min and cutaneous allodynia was assessed over a 5h time period following BLS.
C. On day 32 the mice were again exposed to BLS for 15 min followed by assessment of cutaneous allodynia over a 5h time period.

### Experiment 7 (outline of experiment):

DYSPORT® (0.5 U/mouse) was administered at 72h following the mTBI.

A. In these experiments we first confirmed the development of acute allodynia on days 1-3 after mTBI and then we treated the animals with dysport to investigate if acute allodynia can be reversed.
B. At day 15, mice were exposed to environmental stress (bright light stress, BLS) for 15 min and cutaneous allodynia was assessed over a 5h time period following BLS.

### Results of experiments 1-7

### Experiment 1 (results)

Exp 1A-C (Figures 2, 3, 4): Three independent experiments were performed to demonstrate that DYSPORT® administration at 2 h post mTBI significantly reduces mTBI-induced allodynia and also prevents BLS-induced allodynia at 2-4 weeks post mTBI (Figures 2, 3, 4). The results of all three experiments are consistent and each experiment shows statistically significant effects of mTBI on allodynia as well as statistically significant reduction of allodynia with DYSPORT® treatment. Significant effects of mTBI and reduction by DYSPORT® were also observed at 2 weeks (Figures 2, 3, 4) and 4 weeks (Figures 2, 3) after mTBI in stress-induced allodynia. The findings demonstrate that one administration of DYSPORT® early after a mTBI event prevents mTBI-iduced pain as well as prevent central sensitization that leads to chronification of pain and development of persistent post-traumatic headache.
Exp 1D (Figure 5): We did not detect significant CGRP release in mTBI mice at 4 h and 24 h after mTBI, thus, the effects of DYSPORT® on CGRP levels were not evaluated (Figure 5 A). However, our pilot studies in mice exposed to BLS on day 14 show that BLS promotes CGRP release at 4 h (but not 30 min) post BLS (Figures 5 B, C). We therefore evaluated if DYSPORT® administered at 2 h post mTBI could prevent BLS-induced CGRP release (Figure 5 D). The results of this study show that stress (BLS) leads to increased CGRP blood levels in mTBI, but not sham, mice and DYSPORT® administration at 2 h post mTBI may prevent the CGRP increase.

### Experiment 2 (results)

Exp 2A-C (Figure 6): Figure 6 shows the experiments with DYSPORT® administered 24 h post mTBI. Im portantly, we demonstrated development of both periorbital and hindpaw allodynia at 24 h post mTBI prior to DYSPORT® or saline administration. Subsequent DYSPORT® administration resulted in significant reduction of both periorbital and hindpaw allodynia in DYSPORT®- but not saline- treated mTBI mice. The beneficial effects of DYSPORT® were also evident on day 13, when all mice were exposed to bright lights. While BLS reinstated allodynia in saline treated mTBI mice, DYSPORT® treated mTBI mice had significantly diminished allodynia. Re-exposure of the mice to another BLS episode again on day 17 resulted in periorbital and hindpaw allodynia in saline treated mTBI mice. DYSPORT® treated mice showed reduced periorbital, but not hindpaw allodynia. The findings demonstrate that the window of the efficacy of DYSPORT® in preventing mTBI-induced allodynia extends at least 24 h after a mTBI event (in mice). Thus, one administration of DYSPORT® 24 h after mTBI can be used to prevent mTBI-iduced pain as well as prevent central sensitization that leads to chronification of pain and development of persistent post-traumatic headache.

## Experiment 3:

Exp 3A-C (Figure 7): The experiment 3 was designed to evaluate if DYSPORT® can block BLS-induced allodynia when administered on day 11, 3 days (72 h) before BLS.

Following mTBI or sham injury, mTBI animals developed allodynia that resolved by day 11. DYSPORT® was then administered on day 11 post mTBI and 72 hours before BLS. DYSPORT® prevented latent sensitization revealed by BLS-induced periorbital allodynia. The sham and mTBI animals that previously received saline were given DYSPORT® on day 25 and were again exposed to BLS on day 27.

Overall, results of the experiment 3 demonstate that even after initial acute mTBI-induced allodynia subsides, DYSPORT® is effective in blocking stress-induced allodynia. Thus, DYSPORT® can be used to be effective in patients with persistent post-traumatic headache.

Results of the experiment 3 demonstrate that even after initial acute mTBI-induced allodynia subsides, DYSPORT® is effective in blocking stress-induced allodynia. Thus, DYSPORT® is effective in subjects with persistent post-traumatic headache.

## Experiment 5 (results)

Exp. 5 (Figure 8): DYSPORT® was administered 3 days (72 hours) prior to mTBI to establish a possible efficacy of DYSPORT® as a preventive treatment during activities where mTBI injuries are likely, including competitive sports or military mTBI injuries (e.g., falls). Figure 11 shows that DYSPORT® administered 72 hours before mTBI effectively prevented both acute mTBI-induced allodynia and latent BLS-induced allodynia. Thus, DYSPORT® can be used several days prior to a high risk event to prevent the consequences of a potential head injury.

## Experiment 7 (results)

Exp 6 (Figure 9): To determine if the efficacy window may be extended even beyond 24 h after mTBI, we performed another experiment when DYSPORT® was administered 72 h post mTBI. Development of both periorbital and hindpaw allodynia at 1 day and 3 days post mTBI was verified. Then, DYSPORT® or saline were administered. Compared to saline-treated mTBI mice, DYSPORT®-treated mTBI mice showed significant reduction in periorbital but not hindpaw allodynia. Similarly, following the exposure to BLS, DYSPORT® treated mice showed reduced periorbital but not hindpaw allodynia. Thus, the DYSPORT® efficacy window for the treatment of acute allodynia and stress-induced allodynia is extended up to 72 h post mTBI. Together with the results of Exp. 3 (DYSPORT® administration an day 11 at 72 h before BLS), these results suggest that central sensitization develops 3 days after mTBI resulting in all body allodynia and stress-induced allodynia. However, DYSPORT® is able to block periorbital allodynia.

## EXAMPLE 2 - Further tests of BoNT to treat a symptom of TBI

The efficacy of botulinum neurotoxin, when administered immediately after mild traumatic brain injury (mTBI), in treating the mTBI was evaluated in mice.

The mice were subjected to either mTBI or sham injury on day 0. DYSPORT® (resuspended in 0.9% NaCl from a lyophilisate) was infused through the sagittal suture of each mouse at 0.5 U/mouse immediately following injury.

Facial allodynia and hindpaw allodynia were measured 0, 3, 5, 7, 10, 12, and 14 days post-injury. On day 15, all mice were exposed to bright light stress (BLS) for 15 minutes and facial allodynia and hindpaw allodynia were measured for 5 hours following BLS. On day 32, all mice were exposed again to BLS for 15 minutes and facial allodynia and hindpaw allodynia were again measured for 5 hours following BLS. The results are depicted in Figure 10.

The treatment prevented the onset of acute cephalic and hindpaw allodynia and caused a long-term prevention of sensitization to bright light stress-induced allodynia.

A single mTBI injury resulted in time dependent tactile facial and hindpaw allodynia after mTBI. The administration of 0.5 U DYSPORT® 24 hours immediately following mTBI reversed tactile facial and hindpaw allodynia. Exposure to BLS on Day 15 post-mTBI promoted tactile allodynia in mTBI-injured mice but not sham-injured mice. Allodynia was significantly attenuated in mTBI-injured mice treated with 0.5 U DYSPORT® immediately after mTBI. The second exposure of the mice to BLS promoted tactile allodynia in mTBI-injured mice but not in sham-injured mice. Facial and hindpaw allodynia were significantly attenuated in mTBI-injured mice treated with 0.5 U DYSPORT®.

DYSPORT® administered at immediately following TBI reverses acute TBI-induced allodynia as well as stress-induced allodynia in TBI-primed animals.

## EXAMPLE 3 - Further tests of BoNT to treat a symptom of TBI

The efficacy of botulinum neurotoxin, when administered two hours after mild traumatic brain injury (mTBI), in treating the mTBI was evaluated in mice.

The mice were subjected to either mTBI or sham injury on day 0.

DYSPORT® was infused through the sagittal suture of each mouse at 0.5 U/mouse two hours following injury.

Facial allodynia and hindpaw allodynia were measured 1, 2, 3, 8, 10, 11, and 15 days post-injury. On day 15, all mice were exposed to bright light stress (BLS) for 15 minutes and facial allodynia and hindpaw allodynia were measured for 5 hours following BLS. On day 28, all mice were exposed again to BLS for 15 minutes and facial allodynia and hindpaw allodynia were again measured for 3 hours following BLS. The results are depicted in Figure 11.

A single mTBI injury resulted in time dependent tactile facial and hindpaw allodynia after mTBI. The administration of 0.5 U DYSPORT® 2 hours immediately following mTBI reversed tactile facial and hindpaw allodynia. Exposure to BLS on Day 15 post-mTBI promoted tactile allodynia in mTBI-injured mice but not sham-injured mice. Allodynia was significantly attenuated in mTBI-injured mice treated with 0.5 U DYSPORT® 2 hours after mTBI. The second exposure of the mice to BLS promoted tactile allodynia in mTBI-injured mice but not in sham-injured mice. Facial and hindpaw allodynia were significantly attenuated in mTBI-injured mice treated with 0.5 U DYSPORT®.

DYSPORT® administered 2 hours after TBI reverses acute TBI-induced allodynia as well as stress-induced allodynia in TBI-primed animals.

Levels of CGRP 2 and 3 hours following exposure to BLS on day 28 following injury were measured. The results are shown in Figure 12. The levels of CGRP were increased in untreated mice that suffered mTBI injury as compared with sham injured mice. However, levels were reduced in mTBI-injured mice that were treated with DYSPORT® 2 hours following injury.

## EXAMPLE 4 - Further tests of BoNT to treat a symptom of TBI

The efficacy of botulinum neurotoxin, when administered 24 hours after mild traumatic brain injury (mTBI), in treating the mTBI was evaluated in mice.

The mice were subjected to either mTBI or sham injury on day 0.

On day 1, facial and hindpaw allodynia were assessed. After that, and 24 hours after injury, DYSPORT® was infused through the sagittal suture of each mouse at 0.5 U/mouse.

Facial allodynia and hindpaw allodynia were measured 2, 3, 8, and 13 days post-injury. On day 13, all mice were exposed to bright light stress (BLS) for 15 minutes and facial allodynia and hindpaw allodynia were measured for 5 hours following BLS. On day 17, all mice were exposed again to BLS for 15 minutes and facial allodynia and hindpaw allodynia were again measured for 5 hours following BLS. The results are depicted in Figure 13.

As in Examples 1 and 2, a single mTBI injury resulted in time dependent tactile facial and hindpaw allodynia as demonstrated on day 1 after mTBI and returning to baseline levels by day 13 post-mTBI. The administration of 0.5 U DYSPORT® 24 hours after mTBI reversed tactile facial and hindpaw allodynia. Exposure to BLS on Day 13 post-mTBI promoted tactile allodynia in mTBI-injured mice but not sham-injured mice. Allodynia was significantly attenuated in mTBI-injured mice treated with 0.5 U DYSPORT® 24 hours after mTBI. The second exposure of the mice to BLS promoted tactile allodynia in mTBI-injured mice but not in sham-injured mice. Facial allodynia was significantly attenuated in mTBI-injured mice treated with 0.5 U DYSPORT®. Hindpaw allodynia was reduced in mTBI-injured mice treated with DYSPORT®, but this reduction was not statistically significant.

DYSPORT® administered at 24 hours post TBI reverses acute TBI-induced allodynia as well as stress-induced allodynia in TBI-primed animals.

## EXAMPLE 5 - Further tests of BoNT to treat a symptom of TBI

2.5 mL of preservative-free 0.9% sodium chloride solution is drawn into a syringe and then injected into a vial containing 500 units of DYSPORT®. The vial is tilted side-to-side (and not shaken) for approximately one minute to ensure solution homogeneity.

Using a second syringe, 0.5 mL of the reconstituted DYSPORT® is drawn up without inverting the vial.

2.0 mL of preservative-free 0.9% sodium chloride solution is drawn into a third syringe. The third syringe is then connected to the second syringe with a connector. The plunger of the second syringe is pulled to transfer the 2.0 mL of preservative-free 0.9% sodium chloride solution into the second syringe. The syringe now contains 2.5 mL of solution containing 100 units of DYSPORT®.

A 1 mL syringe is then connected using a connector to the syringe containing the DYSPORT® solution. The plunger of the 1 mL syringe is pulled to transfer 0.8 mL of the DYSPORT® solution to the 1 mL solution. The syringe is removed and capped.

The solution is stored in the syringe in a refrigerator at 2 to 8 °C and protected from light. The composition is used within 24 hours and, if not used, is discarded. Before use in injection, the cap is removed and a needle attached.

## EXAMPLE 6 - Further tests of BoNT to treat a symptom of TBI

A patient suffering from a blow to the head is immediately seen by a physician.

The physician measures the severity of the patient's injury using the Glasgow Coma Scale (GCS). The patient's GCS score is determined to be 6 and thus severe.

The likelihood of the patient developing a symptom of TBI is then determined by measuring the level of UCH-L1 in his blood with higher than normal amounts indicating a high likelihood that he would develop a symptom of TBI.

Higher than normal amounts were measured and thus a higher dosage amount of 25 units/kg per treatment session is determined to be appropriate.

The weight of the patient is determined to be 80 kg. As such, 2,000 units of Dysport® are administered within an hour of the TBI.

The patient's response to the administration is then assessed using Rancho Los Amigos scale after administration. The patient's response exhibits a Level VIII response. As such, administration of botulinum neurotoxin is discontinued.

## EXAMPLE 7 - Further tests of BoNT to treat a symptom of TBI

A patient that has lost consciousness after a fall is examined by a physician.

The patient regains consciousness after 3 hours. As LOC was 3 hours, the patient has suffered a moderated case of TBI.

The likelihood of the patient developing a symptom of TBI is then determined by measuring the level of GFAP in his blood. The level is determined to be only slightly above normal, indicating a moderate likelihood that he would develop a symptom of TBI.

In view of the above, a moderate dosage amount of 15 units/kg per treatment session is determined to be appropriate.

The weight of the patient is determined to be 60 kg. As such, 900 units of Dysport® are administered (20 hours after TBI).

The patient's response to the administration is then assessed using the Rancho Los Amigos scale. The patient's response exhibits a Level VI response. As such, a further administration is scheduled but at a lower dose of 10 units/kg.

## EXAMPLE 8 - Further tests of BoNT to treat a symptom of TBI

A patient that has suffered from post-traumatic amnesia (PTA) after being a car accident is examined by a physician.

The patient regains continuous memory after 15 minutes. As PTA lasted less than 20 minutes, the patient has suffered a mild case of TBI.

The likelihood of the patient developing a symptom of TBI is then determined by measuring the level of CGRP in her blood. The level is determined to be normal, indicating a low likelihood that she would develop a symptom of TBI.

In view of the above, a low dosage amount of 5 units/kg per treatment session is determined to be appropriate.

The weight of the patient is determined to be 50 kg. As such, 250 units of Dysport® are administered (10 hours after TBI).

The patient's response to the administration is then assessed using Rancho Los Amigos scale. The patient's response exhibits a Level IV response. As such, a further administration of botulinum neurotoxin is scheduled but at a higher dose of 10 units/kg per treatment session.

## Claims

**1.** A composition for use in a method of treating a traumatic brain injury-induced symptom in a patient suffering a traumatic brain injury (TBI), the composition comprising a therapeutically-effective amount of a botulinum neurotoxin (BoNT),

wherein the composition is administered within 72 hours following injury, and/or
wherein the composition is administered up to 4 days pre-injury;

wherein the TBI-induced symptom is TBI-induced pain and/or TBI-induced light sensitivity.

2. The composition for use according to claim 1, wherein the administration is local administration to the subject's head.

3. The composition for use according to claim 1 or claim 2, wherein the botulinum neurotoxin is administered in a single dose; and/or
wherein the botulinum neurotoxin is administered subcutaneously; and/or wherein the amount of botulinum neurotoxin administered is between 20-70 units, optionally wherein the amount of botulinum neurotoxin administered is 50 units.

4. The composition for use according to any one of the preceding claims, wherein the botulinum neurotoxin is administered between 1-3 days pre-injury; optionally wherein the botulinum neurotoxin is administered 3 days pre-injury.

5. The composition for use according to any one of the preceding claims, wherein the botulinum neurotoxin is administered within 48 hours following injury; and/or wherein the botulinum neurotoxin is administered within 2 hours following injury; and/or wherein the botulinum neurotoxin is administered immediately following injury.

6. The composition for use according to any one of the preceding claims, wherein the botulinum neurotoxin is administered at least an hour following injury.

7. The composition for use according to any one of the preceding claims, wherein:
the botulinum neurotoxin is administered 2 to 72 hours following injury.

8. The composition for use according to any one of the preceding claims, wherein:

(a) the amount of botulinum neurotoxin administered is less than 30 units per kilogram of the total body weight of the subject;
(b) the botulinum neurotoxin is administered parenterally;
(c) the botulinum neurotoxin is administered through a suture of the skull of the subject;
(d) the botulinum neurotoxin is: botulinum neurotoxin type A; botulinum neurotoxin type A1; botulinum neurotoxin type A2; botulinum neurotoxin type A3; botulinum neurotoxin type A4; botulinum neurotoxin type B; botulinum neurotoxin type C; botulinum neurotoxin type C1; botulinum neurotoxin type D; botulinum neurotoxin type E; botulinum neurotoxin type F; or botulinum neurotoxin type G, optionally wherein the botulinum neurotoxin is botulinum neurotoxin type A;
(e) the method further comprises measuring the severity of the symptom of the traumatic brain injury, optionally wherein the amount of botulinum neurotoxin administered is determined based on the level of severity of the symptom of traumatic brain injury;
(f) the method further comprises measuring the severity of the traumatic brain injury, optionally wherein the amount of botulinum neurotoxin administered is determined based on the level of severity of the traumatic brain injury;
(g) the amount of botulinum neurotoxin administered is determined based on the weight of the subject;
(h) the amount of botulinum neurotoxin administered is determined based on whether the subject has previously experienced traumatic brain injury;
(i) the amount of botulinum neurotoxin administered is determined based on the amount of time that has elapsed following injury;
(j) the composition further comprises a diluent, optionally wherein the diluent comprises a lyophilized powder, and optionally wherein the composition is formed by reconstituting lyophilized powder comprising botulinum neurotoxin; and/or
(k) the composition further comprises an excipient, optionally wherein the excipient is: a filler, a binder, a disintegrant, an anti-adherent, a solvent, a buffering agent, a preservative, or a humectant.

9. The composition for use according to any one of the preceding claims, the method further comprising:

   (a) measuring the subject's response to the administration of botulinum toxin; and/or
   (b) measuring a dosing regimen for botulinum neurotoxin based on the subject's response to a previous administration thereof, optionally wherein a subsequent administration of botulinum neurotoxin is based on the dosing regimen determined.

10. The composition for use according to any one of the preceding claims, further comprising recording the subject's response to an administration of botulinum neurotoxin, optionally wherein the subject's response is recorded on a first computer device and is accessed on a second computer device; preferably
    wherein the subject's response is recorded into a software program that is configured to receive the information, and/or further comprising storing the subject's response in a database.

11. The composition for use according to any one of the preceding claims, the method further comprising determining whether the subject has a symptom of traumatic brain injury before administering the botulinum neurotoxin; preferably wherein the step of determining whether the subject has a symptom of traumatic brain injury comprises measuring the level of a biomarker in the blood of the subject, the level being indicative of whether the subject has or is likely to develop the symptoms of traumatic brain injury, optionally wherein the biomarker is: ubiquitin carboxy-terminal hydrolase L1, glial fibrillary acidic protein and calcitonin gene related peptide, TNF-$\alpha$, or NF-L.

**Patentansprüche**

1. Eine Zusammensetzung zur Verwendung in einem Verfahren zum Behandeln eines durch ein Schädelhirntrauma induzierten Symptoms bei einem Patienten, der ein Schädelhirntrauma (SHT) erleidet , wobei die Zusammensetzung eine therapeutisch wirksame Menge eines Botulinumneurotoxins (BoNT) umfasst,

   wobei die Zusammensetzung innerhalb von 72 Stunden nach der Schädigung verabreicht wird und/oder
   wobei die Zusammensetzung bis zu 4 Tage vor der Schädigung verabreicht wird;
   wobei das SHT-induzierte Symptom SHT-induzierte Schmerzen und/oder SHT-induzierte Lichtempfindlichkeit ist.

2. Zusammensetzung zur Verwendung nach Anspruch 1, wobei die Verabreichung eine lokale Verabreichung in den Kopf des Individuums ist.

3. Zusammensetzung zur Verwendung nach Anspruch 1 oder Anspruch 2, wobei das Botulinumneurotoxin in einer Einzeldosis verabreicht wird; und/oder
   wobei das Botulinumneurotoxin subkutan verabreicht wird; und/oder wobei die verabreichte Botulinumneurotoxinmenge zwischen 20-70 Einheiten beträgt, wobei die verabreichte Botulinumneurotoxinmenge optional 50 Einheiten beträgt.

4. Zusammensetzung zur Verwendung nach einem der vorhergehenden Ansprüche, wobei das Botulinumneurotoxin zwischen 1-3 Tage vor der Schädigung verabreicht wird; wobei das Botulinumneurotoxin optional 3 Tage vor der Schädigung verabreicht wird.

5. Zusammensetzung zur Verwendung nach einem der vorhergehenden Ansprüche, wobei das Botulinumneurotoxin innerhalb von 48 Stunden nach der Schädigung verabreicht wird; und/oder wobei das Botulinumneurotoxin innerhalb von 2 Stunden nach der Schädigung verabreicht wird; und/oder wobei das Botulinumneurotoxin unmittelbar nach der Schädigung verabreicht wird.

6. Zusammensetzung zur Verwendung nach einem der vorhergehenden Ansprüche, wobei das Botulinumneurotoxin mindestens eine Stunde nach der Schädigung verabreicht wird.

7. Zusammensetzung zur Verwendung nach einem der vorhergehenden Ansprüche, wobei:
   das Botulinumneurotoxin 2 bis 72 Stunden nach der Schädigung verabreicht wird.

8. Zusammensetzung zur Verwendung nach einem der vorhergehenden Ansprüche, wobei:

(a) die verabreichte Botulinumneurotoxinmenge weniger als 30 Einheiten pro Kilogramm des Gesamtkörpergewichts des Individuums beträgt;

(b) das Botulinumneurotoxin parenteral verabreicht wird;

(c) das Botulinumneurotoxin durch eine Naht des Schädels des Individuums verabreicht wird;

(d) das Botulinumneurotoxin Folgendes ist:

Botulinumneurotoxin Typ A; Botulinumneurotoxin Typ A1;
Botulinumneurotoxin Typ A2; Botulinumneurotoxin Typ A3;
Botulinumneurotoxin Typ A4; Botulinumneurotoxin Typ B;
Botulinumneurotoxin Typ C; Botulinumneurotoxin Typ C1;
Botulinumneurotoxin Typ D; Botulinumneurotoxin Typ E;
Botulinumneurotoxin Typ F; oder Botulinumneurotoxin Typ G,
wobei das Botulinumneurotoxin optional Botulinumneurotoxin Typ A ist;

(e) das Verfahren ferner das Messen der Schwere des Symptoms des Schädelhirntraumas umfasst, wobei die verabreichte Botulinumneurotoxinmenge optional auf der Grundlage des Schweregrads des Schädelhirntraumasymptoms bestimmt wird;

(f) das Verfahren ferner das Messen der Schwere des Schädelhirntraumas umfasst, wobei die verabreichte Botulinumneurotoxinmenge optional auf der Grundlage des Schweregrads des Schädelhirntraumas bestimmt wird;

(g) die verabreichte Botulinumneurotoxinmenge auf der Grundlage des Gewichts des Individuums bestimmt wird;

(h) die verabreichte Botulinumneurotoxinmenge aufgrund dessen bestimmt wird, ob es bei dem Individuum zuvor zu einem Schädelhirntrauma gekommen war;

(i) die verabreichte Botulinumneurotoxinmenge auf der Grundlage der nach der Schädigung verstrichenen Zeitspanne bestimmt wird;

(j) die Zusammensetzung ferner ein Verdünnungsmittel umfasst, wobei das Verdünnungsmittel optional ein lyophilisiertes Pulver umfasst, und wobei die Zusammensetzung optional durch Rekonstituieren des lyophilisierten Pulvers, das Botulinumneurotoxin umfasst, gebildet wird; und/oder

(k) die Zusammensetzung ferner einen Hilfsstoff umfasst, wobei der Hilfsstoff optional Folgendes ist: ein Füllmittel, ein Bindemittel, ein Sprengmittel, ein Antiadhäsionsmittel, ein Lösemittel, ein Puffermittel, ein Konservierungsmittel oder ein Feuchthaltemittel.

9. Zusammensetzung zur Verwendung nach einem der vorhergehenden Ansprüche, wobei das Verfahren ferner Folgendes umfasst:

(a) Messen des Ansprechens des Individuums auf die Verabreichung von Botulinumtoxin; und/oder

(b) Messen eines Behandlungsschemas für Botulinumneurotoxin auf der Grundlage des Ansprechens des Individuums auf eine vorherige Verabreichung davon, wobei eine nachfolgende Verabreichung von Botulinumneurotoxin optional auf dem bestimmten Behandlungsschema basiert.

10. Zusammensetzung zur Verwendung nach einem der vorhergehenden Ansprüche, die ferner das Aufzeichnen des Ansprechens des Individuums auf eine Verabreichung von Botulinumneurotoxin umfasst, wobei das Ansprechen des Individuums optional auf einem ersten Computergerät aufgezeichnet wird und der Zugriff darauf auf einem zweiten Computergerät erfolgt;

wobei das Ansprechen des Individuums bevorzugt in einem Softwareprogramm aufgezeichnet wird, das dazu ausgelegt ist, die Informationen zu erhalten, und/oder ferner das Speichern des Ansprechens des Individuums in einer Datenbank umfasst.

11. Zusammensetzung zur Verwendung nach einem der vorhergehenden Ansprüche, wobei das Verfahren ferner das Bestimmen umfasst, ob das Individuum ein Schädelhirntraumasymptom aufweist, bevor das Botulinumneurotoxin verabreicht wird; bevorzugt

wobei der Schritt des Bestimmens, ob das Individuum ein Schädelhirntraumasymptom aufweist, das Messen der Konzentration eines Biomarkers in dem Blut des Individuums umfasst, wobei die Konzentration darauf hinweist, ob das Individuum die Schädelhirntraumasymptome aufweist oder diese wahrscheinlich auftreten werden, wobei der Biomarker optional Folgendes ist: Ubiquitin carboxy-terminal hydrolase L1, Glial fibrillary acidic protein und Calcitonin gene related peptide, TNF-$\alpha$ oder NF-L.

**Revendications**

1. Composition destinée à une utilisation dans une méthode de traitement d'un symptôme induit par une lésion cérébrale traumatique chez un patient souffrant d'une lésion cérébrale traumatique (LCT), la composition comprenant une quantité thérapeutiquement efficace d'une neurotoxine botulique (BoNT),

   la composition étant administrée dans les 72 heures suivant la lésion, et/ou
   la composition étant administrée jusqu'à 4 jours avant la lésion ;
   le symptôme induit par la LCT étant une douleur induite par la LCT et/ou une sensibilité à la lumière induite par la LCT.

2. Composition destinée à une utilisation selon la revendication 1, l'administration étant une administration locale au niveau de la tête du sujet.

3. Composition destinée à une utilisation selon la revendication 1 ou la revendication 2, la neurotoxine botulique étant administrée en une seule dose ; et/ou
   la neurotoxine botulique étant administrée par voie sous-cutanée ; et/ou la quantité de neurotoxine botulique administrée étant comprise entre 20 et 70 unités, optionnellement, la quantité de neurotoxine botulique administrée étant de 50 unités.

4. Composition destinée à une utilisation selon l'une quelconque des revendications précédentes, la neurotoxine botulique étant administrée entre 1 et 3 jours avant la lésion ; optionnellement, la neurotoxine botulique étant administrée 3 jours avant la lésion.

5. Composition destinée à une utilisation selon l'une quelconque des revendications précédentes, la neurotoxine botulique étant administrée dans les 48 heures suivant la lésion ; et/ou la neurotoxine botulique étant administrée dans les 2 heures suivant la lésion ; et/ou la neurotoxine botulique étant administrée immédiatement après la lésion.

6. Composition destinée à une utilisation selon l'une quelconque des revendications précédentes, la neurotoxine botulique étant administrée au moins une heure après la lésion.

7. Composition destinée à une utilisation selon l'une quelconque des revendications précédentes,
   la neurotoxine botulique étant administrée 2 à 72 heures après la lésion.

8. Composition destinée à une utilisation selon l'une quelconque des revendications précédentes,

   (a) la quantité de neurotoxine botulique administrée étant inférieure à 30 unités par kilogramme du poids corporel total du sujet ;
   (b) la neurotoxine botulique étant administrée par voie parentérale ;
   (c) la neurotoxine botulique étant administrée par une suture du crâne du sujet ;
   (d) la neurotoxine botulique étant : une neurotoxine botulique de type A ; une neurotoxine botulique de type A1 ; une neurotoxine botulique de type A2 ; une neurotoxine botulique de type A3 ; une neurotoxine botulique de type A4 ; une neurotoxine botulique de type B ; une neurotoxine botulique de type C ; une neurotoxine botulique de type C1 ; une neurotoxine botulique de type D ; une neurotoxine botulique de type E ; une neurotoxine botulique de type F ; ou une neurotoxine botulique de type G, optionnellement, la neurotoxine botulique étant une neurotoxine botulique de type A ;
   (e) la méthode comprenant en outre une mesure de la sévérité du symptôme de la lésion cérébrale traumatique, optionnellement, la quantité de neurotoxine botulique administrée étant déterminée en fonction du niveau de sévérité du symptôme de lésion cérébrale traumatique ;
   (f) la méthode comprenant en outre une mesure de la sévérité de la lésion cérébrale traumatique, optionnellement, la quantité de neurotoxine botulique administrée étant déterminée en fonction du niveau de sévérité de la lésion cérébrale traumatique ;
   (g) la quantité de neurotoxine botulique administrée étant déterminée en fonction du poids du sujet ;
   (h) la quantité de neurotoxine botulique administrée étant déterminée selon que le sujet a ou non subi auparavant une lésion cérébrale traumatique ;
   (i) la quantité de neurotoxine botulique administrée étant déterminée en fonction du temps qui s'est écoulé depuis la lésion ;
   (j) la composition comprenant en outre un diluant, optionnellement, le diluant comprenant une poudre lyophilisée, et optionnellement, la composition étant formée par reconstitution d'une poudre lyophilisée comprenant une neurotoxine botulique ; et/ou
(k) la composition comprenant en outre un excipient, optionnellement, l'excipient étant : une charge, un liant, un délitant, un antiadhérent, un solvant, un agent tampon, un conservateur ou un humectant.

9. Composition destinée à une utilisation selon l'une quelconque des revendications précédentes, la méthode comprenant en outre :

(a) une mesure de la réponse du sujet à l'administration de toxine botulique ; et/ou
(b) une mesure d'un schéma posologique de la neurotoxine botulique en fonction de la réponse du sujet à une administration antérieure de celle-ci, optionnellement, une administration ultérieure de neurotoxine botulique étant basée sur le schéma posologique déterminé.

10. Composition destinée à une utilisation selon l'une quelconque des revendications précédentes, comprenant en outre un enregistrement de la réponse du sujet à une administration de neurotoxine botulique, optionnellement, la réponse du sujet étant enregistrée sur un premier dispositif informatique et étant accessible sur un deuxième dispositif informatique ; de préférence, la réponse du sujet étant enregistrée dans un logiciel qui est configuré pour recevoir l'information, et/ou comprenant en outre un stockage de la réponse du sujet dans une base de données.

11. Composition destinée à une utilisation selon l'une quelconque des revendications précédentes, la méthode comprenant en outre le fait de déterminer si le sujet présente un symptôme de lésion cérébrale traumatique avant l'administration de la neurotoxine botulique ; de préférence, l'étape consistant à déterminer si le sujet présente un symptôme de lésion cérébrale traumatique comprenant une mesure du niveau d'un biomarqueur dans le sang du sujet, le niveau indiquant si le sujet présente ou est susceptible de développer les symptômes d'une lésion cérébrale traumatique, optionnellement, le biomarqueur étant : l'hydrolase carboxy-terminale de l'ubiquitine L1, la protéine gliofibrillaire acide et le peptide lié au gène de la calcitonine, le TNF-$\alpha$ ou le NF-L.

FIGURE 1

FIGURE 2

Dysport administered 2 h after mTBI

Dysport administered 2 h after mTBI

A — Facial allodynia (following mTBI)
B — Facial allodynia (response to BLS, D14/15)
C — Facial allodynia (response to BLS, D26/27)
D — Hindpaw allodynia (following mTBI)
E — Hindpaw allodynia (response to BLS, D14/15)
F — Hindpaw allodynia (response to BLS, D26/27)

FIGURE 3

EP 3 890 772 B1

FIGURE 4

# Dysport administered 2 h after mTBI

FIGURE 5

# CGRP blood levels

**A** Blood CGRP levels
(after mTBI)

**B** Blood CGRP levels
(D14 after mTBI; 30 min after BLS)

**C** Blood CGRP levels
(D14 after mTBI; 4 h after BLS)

**D** Blood CGRP levels
(D28 after mTBI; 2 or 3 h after BLS)

**Dysport administered 24 h after mTBI**

A — Facial allodynia (following mTBI)

B — Facial allodynia (response to BLS, D13)

C — Facial allodynia (response to BLS, D17)

D — Hindpaw allodynia (following mTBI)

E — Hindpaw allodynia (response to BLS, D13)

F — Hindpaw allodynia (response to BLS, D17)

EP 3 890 772 B1

FIGURE 6

FIGURE 7

# Dysport administered 72 h before BLS

A
Facial allodynia
(following mTBI)

C
Facial allodynia
(response to BLS, D14)

B
Hindpaw allodynia
(following mTBI)

D
Hindpaw allodynia
(response to BLS, D14)

EP 3 890 772 B1

FIGURE 8

# Dysport administered 72 h before mTBI

A — Facial allodynia (following mTBI)

B — Facial allodynia (response to BLS, D12)

C — Hindpaw allodynia (following mTBI)

D — Hindpaw allodynia (response to BLS, D12)

40

FIGURE 9

# Dysport administered 72 h after mTBI

**A** Facial allodynia (following mTBI)

**B** Facial allodynia (response to BLS, D14/15)

**C** Hindpaw allodynia (following mTBI)

**D** Hindpaw allodynia (response to BLS, D14/15)

FIGURE 10
A

B

FIGURE 10 (continued)

C

D

FIGURE 10 (continued)

E

F

FIGURE 11

A

B

FIGURE 11 (continued)

C

D

FIGURE 11 (continued)

E

F

FIGURE 12

*FIG. 3*

FIGURE 13
A

B

FIGURE 13 (continued)

C

D

FIGURE 13 (continued)

E

F

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006078588 A2
- WO 99032272 A
- WO 2011022357 A
- WO 9421300 A
- WO 9633273 A
- WO 9807864 A
- WO 2011018665 A
- US 20070166332 A
- WO 2007104567 A
- WO 08008803 A
- WO 08008805 A
- WO 2015004461 A
- WO 2015166242 A
- WO 2016110662 A
- WO 2006114308 A
- WO 2013180799 A
- WO 2017191315 A
- US 5223409 A, Ladner
- WO 9206204 A, Huse

**Non-patent literature cited in the description**

- **LEWIS et al.** *Acad Emerg Med*, 2017, vol. 24 (6), 710-720
- **BREE** ; **LEVY**. *Cephalalgia*, 2018, vol. 38, 246-258
- **MACKENZIE et al.** *PM R.*, July 2015, vol. 7 (7), 785-788
- **PATIL et al.** *Curr Pain Headache Rep*, March 2016, vol. 20 (3), 15
- **PARK** ; **CHUNG**. *Toxins*, 01 June 2018, vol. 10 (6), 224
- **LIPPERT-GRÜNER**. *Neurol Neurochir Pol*, November 2012, vol. 46 (6), 591-4
- **GERALD K**. Cell and Molecular Biology. John Wiley & Sons, Inc, 2002
- **CHEN** ; **BARBIERI**. *PNAS*, 2009, vol. 106 (23), 9180-9184
- **HENDERSON et al.** The Clostridia: Molecular Biology and Pathogenesis. Academic press, 1997
- **O'KEEFE et al.** *Proc. Natl. Acad. Sci. USA*, 1992, vol. 89, 6202-6206
- **SILVERMAN et al.** *J. Biol. Chem.*, 1993, vol. 269, 22524-22532
- **LONDON, E.** *Biochem. Biophys. Acta.*, 1992, vol. 1112, 25-51
- **PRIOR et al.** *Biochemistry*, 1992, vol. 31, 3555-3559
- **BLANKE et al.** *Proc. Natl. Acad. Sci. USA*, 1996, vol. 93, 8437-8442
- **PLANK et al.** *J. Biol. Chem.*, 1994, vol. 269, 12918-12924
- **WAGNER et al.** *PNAS*, 1992, vol. 89, 7934-7938
- **MURATA et al.** *Biochem.*, 1992, vol. 31, 1986-1992
- **SIMPSON D.M. et al.** Assessment: Botulinum neurotoxin for the treatment of spasticity (an evidence-based review): Report of the Therapeutics and Technology Assessment Subcommittee of the American Academy of Neurology. *Neurology 6*, 2008, vol. 70 (19), 1691-1698
- **CUI et al.** *Pain*, 2004, vol. 107, 125-133
- **MARINO et al.** *Pain*, 2014, vol. 155, 674-684
- **CERNUDA-MOROLLON et al.** *Pain*, 2015, vol. 156, 820-824
- **LEE et al.** *Anesth Analg*, 2011, vol. 112, 228-235
- **JULIE D. THOMPSON et al.** CLUSTAL W: Improving the Sensitivity of Progressive Multiple Sequence Alignment Through Sequence Weighting, Position-Specific Gap Penalties and Weight Matrix Choice. *Nucleic Acids Research*, 1994, vol. 22 (22), 4673-4680
- **OSAMU GOTOH**. Significant Improvement in Accuracy of Multiple Protein. Sequence Alignments by Iterative Refinement as Assessed by Reference to Structural Alignments. *J. Mol. Biol.*, 1996, vol. 264 (4), 823-838
- **ERIC DEPIEREUX** ; **ERNEST FEYTMANS**. Match-Box: A Fundamentally New Algorithm for the Simultaneous Alignment of Several Protein Sequences. *CABIOS*, 1992, vol. 8 (5), 501-509
- **C. E. LAWRENCE et al.** Detecting Subtle Sequence Signals: A Gibbs Sampling Strategy for Multiple Alignment. *Science*, 1993, vol. 262 (5131), 208-214
- **IVO VAN WALLE et al.** Align-M - A New Algorithm for Multiple Alignment of Highly Divergent Sequences. *Bioinformatics*, 2004, vol. 20 (9), 1428-1435

- **ALTSCHUL et al.** *Bull. Math. Bio.*, 1986, vol. 48, 603-16
- **HENIKOFF** ; **HENIKOFF**. *Proc. Natl. Acad. Sci. USA*, 1992, vol. 89, 10915-19
- **ROBERTSON et al.** *J. Am. Chem. Soc.*, 1991, vol. 113, 2722
- **ELLMAN et al.** *Methods Enzymol.*, 1991, vol. 202, 301
- **CHUNG et al.** *Science*, 1993, vol. 259, 806-9
- **CHUNG et al.** *Proc. Natl. Acad. Sci. USA*, 1993, vol. 90, 10145-9
- **TURCATTI et al.** *J. Biol. Chem.*, 1996, vol. 271, 19991-8
- **KOIDE et al.** *Biochem.*, 1994, vol. 33, 7470-6
- **WYNN** ; **RICHARDS**. *Protein Sci.*, 1993, vol. 2, 395-403
- **CUNNINGHAM** ; **WELLS**. *Science*, 1989, vol. 244, 1081-5
- **DE VOS et al.** *Science*, 1992, vol. 255, 306-12
- **SMITH et al.** *J. Mol. Biol.*, 1992, vol. 224, 899-904
- **WLODAVER et al.** *FEBS Lett*, 1992, vol. 309, 59-64
- **REIDHAAR-OLSON** ; **SAUER**. *Science*, 1988, vol. 241, 53-7
- **BOWIE** ; **SAUER**. *Proc. Natl. Acad. Sci. USA*, 1989, vol. 86, 2152-6
- **LOWMAN et al.** *Biochem.*, 1991, vol. 30, 10832-7
- **DERBYSHIRE et al.** *Gene*, 1986, vol. 46, 145
- **NER et al.** *DNA*, 1988, vol. 7, 127
- **SINGLETON et al.** DICTIONARY OF MICROBIOLOGY AND MOLECULAR BIOLOGY. John Wiley and Sons, 1994
- **HALE** ; **MARHAM**. THE HARPER COLLINS DICTIONARY OF BIOLOGY. Harper Perennial, 1991
- **KANE et al.** *J Neurosci Methods*, 2012, vol. 203, 41-49